# EUROPEAN PATENT APPLICATION

(11) **EP 2 279 997 A1**
(43) Date of publication of application: **02.02.2011**
(21) Application number: 09305702.4
(22) Date of filing: 23.07.2009
(51) Int. Cl.: C07C 229/48, C07C 309/19

(54) **Fluorinated cyclopropane analogs of glutamic acid**

(71) Applicant: Institut National des Sciences Appliquées de Rouen (INSA), 76130 Mont Saint Aignan (FR)
(72) Inventor: Jubault, Philippe, 76160 Preaux (FR); Quirion, Jean-Charles, 27310 Bourg-Achard (FR); Lion Cèendric, 59110 La Madeleine (FR); Lemonnier, Gérald, 27210 Berville Sur Mer (FR)
(74) Representative: Warcoin, Jacques

(57) **Abstract**

The present invention relates to a compound of the following formula (I): 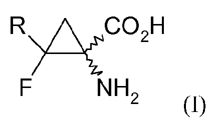 or a pharmaceutically acceptable salt, a stereoisomer or a mixture in all proportions of stereoisomers thereof, in particular a mixture of enantiomers, such as a racemic mixture,
wherein R represents a (C₁-C₆)alkanediyl or (C₁-C₆)alkenediyl group, optionally substituted by one or more groups chosen among an halogen atom, ORₐ, SR_{b}, NR_{c}R_{d}, PO(ORₑ)(OR_{f}), CO₂R_{g}, SO₂Rₕ SO₃Rᵢ, PO(OH)(CH(OH)Rₖ), CN, N₃ and NH-C(=NH)NH₂,
with Rₐ, R_{b}, R_{c} and R_{d}, representing, independently of each other, an hydrogen atom, a (C₁-C₆)alkyl group or a -CO-(C₁-C₆)alkyl group,
Rₑ, R_{f}, R_{g}, Rₕ and Rᵢ representing, independently of each other, an hydrogen atom or a (C₁-C₆)alkyl group, and
Rₖ representing an aryl or heteroaryl group, said group being optionally substituted by one or more groups selected from an halogen atom and NO₂,
as well as to the use thereof and to a process for preparing such a compound, to a pharmaceutical composition containing it and to synthesis intermediates.

## Description

The present invention concerns new fluorinated cyclopropane amino acid derivatives, as well as their use, notably in the treatment of neurological diseases, such as Alzheimer's disease, Parkinson's disease or epilepsy, and a process for preparing such compounds.

The cyclopropane moiety is present in numerous biological active compounds with various activities such as enzyme inhibition, insecticide, antifungal, antibiotic, antitumoral, etc. Indeed, the presence of a cyclopropane moiety can potentially increase selectivity and affinity for biological receptors of the molecule by changing or blocking the spatial organisation of the different radicals of the related molecule.

The presence of a fluorine atom on the cyclopropane ring can further increase the reactivity of the molecule by modulating the acidity and basicity of neighbouring groups, the lipophily, the bound length, and also the electronic distribution, which allows a modulation of the pharmacologic parameters (absorption, distribution, affinity, etc).

However, there exist few methods for preparing such fluorocyclopropane derivatives, and there is no method for preparing fluorinated cyclopropane amino acid derivatives.

The inventors have thus developed a method for preparing such amino acid derivatives bearing a fluorocyclopropane moiety. This process allows to give access to compounds which can be useful as analogues of amino acids (such as leucine, methionine, homocystéine, homosérine, lysine, arginine, etc.) with different physicochemical properties such as stability. Moreover, some of them can be useful in the treatment of neurological diseases, such as Alzheimer's disease, Parkinson's disease or epilepsy, as ligand of metabotropic glutamate receptors (mGluRs), notably as analogues of glutamic acid, L-(+)-2-amino-4-phosphonobutyric acid (L-AP4), or (1*S*,2*S*)-1-amino-2phosphonomethylcyclopropanecarboxylic acid ((+)-(1*S*,2*S*)-APCPr), compounds known as agonists of the metabotropic glutamate receptors.

Thus, the present invention has for first object a compound of the following formula (I): 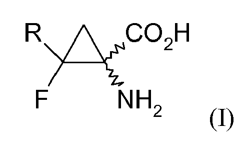 or a pharmaceutically acceptable salt, a stereoisomer or a mixture in all proportions of stereoisomers thereof, in particular a mixture of enantiomers, such as a racemic mixture,
wherein R represents a (C₁-C₆)alkanediyl or (C₁-C₆)alkenediyl group, optionally substituted by one or more groups chosen among an halogen atom, ORₐ, SR_{b}, NR_{c}R_{d}, PO(ORₑ)(OR_{f}), CO₂Rg, SO₂Rₕ SO₃Rᵢ, PO(OH)(CH(OH)Rₖ), CN, N₃ and NH-C(=NH)NH₂,
with Rₐ, R_{b}, R_{c} and R_{d}, representing, independently of each other, an hydrogen atom, a (C₁-C₆)alkyl group or a -CO-(C₁-C₆)alkyl group,
Rₑ, R_{f}, Rg, Rₕ and Rᵢ representing, independently of each other, an hydrogen atom or a (C₁-C₆)alkyl group, and
Rₖ representing an aryl or heteroaryl group, said group being optionally substituted by one or more groups selected from an halogen atom and NO₂.

For the purpose of the invention, the term "pharmaceutically acceptable" is intended to mean which is useful to the preparation of a pharmaceutical composition, and which is generally safe and non toxic, for a pharmaceutical use.

The term "pharmaceutically acceptable salt" is intended to mean, in the framework of the present invention, a salt of a compound which is pharmaceutically acceptable, as defined above, and which possesses the pharmacological activity of the corresponding compound. Such salts comprise:
(1) hydrates and solvates,
(2) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, or
(3) salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

For the purpose of the invention, the term "stereoisomers" is intended to mean diastereoisomers or enantiomers. It corresponds thus to optical isomers. The stereoisomers which are not mirror images of each other, are thus called "diastereoisomers", whereas the stereoisomers which are mirror images of each other but non superimposable are called "enantiomers".

For the purpose of the invention, the term "racemic mixture" is intended to mean a mixture of two enantiomers in equal quantities.

The term "(C₁-C₆)alkanediyl" as used in the present invention refers to a straight or branched divalent saturated hydrocarbon chain containing from 1 to 6 carbon atoms including, but not limited to, methanediyl, ethanediyl, propanediyl, butanediyl, pentanediyl, hexanediyle, and the like.

The term "(C₁-C₆)alkenediyl" as used in the present invention refers to a straight or branched divalent unsaturated hydrocarbon chain containing from 1 to 6 carbon atoms and comprising at least one double bound including, but not limited to, ethenediyle, propenediyl, butenediyl, pentenediyl, hexenediyl and the like.

The term "halogen" as used in the present invention refers to a fluorine, bromine, chlorine or iodine atom.

The term "(C₁-C₆)alkyl" as used in the present invention refers to a straight or branched monovalent saturated hydrocarbon chain containing from 1 to 6 carbon atoms including, but not limited to, methyl, ethyl, n-propyl, iso-propyl, n-butyl, isobutyl, sec-butyl, t-butyl, n-pentyl, n-hexyl and the like.

The term "-CO-(C₁-C₆)alkyl" as used in the present invention refers to a (C₁-C₆)alkyl group as define above bound to the molecule via a carbonyl group (CO). It can be for example an acetyl group.

The term "(C₁-C₆)alkoxy" as used in the present invention refers to a (C₁-C₆)alkyl group as define above bound to the molecule via an oxygen atom. It can be for example a methoxy, ethoxy, n-propoxy, isopropxy or *tert*-buoxy group.

The term "aryl" as used in the present invention refers to an aromatic group comprising preferably 5 to 10 carbon atoms and comprising one or more fused rings, such as, for example, a phenyl or naphtyl group. Advantageously, it will be a phenyl group.

The term "heteroaryl" as used in the present invention refers to an aryl group, as defined above, in which one or more, advantageously 1 to 4, and more advantageously 1 or 2, carbon atoms have been replaced by respectively one or more, advantageously 1 to 4, and more advantageously 1 or 2, heteroatom, such as a nitrogen, oxygen or sulphur atom. An heteroaryl group can be notably thienyl, furanyl, pyrrolyl, etc.

Thus such compounds can be useful as analogues of amino acid derivatives, notably in the synthesis of new peptides or in the treatment of neurological diseases (as analogues of glutamic acid).

According to a particular embodiment of the present invention, R can be a (C₁-C₆)alkanediyl group, such as -CH₂- or -CH₂-CH₂-, substituted by a group chosen among PO₃H₂, CO₂H and SO₃H, and optionally substituted by one or more groups chosen among an halogen atom, ORₐ, SR_{b} and NR_{c}R_{d}, with Rₐ, R_{b}, R_{c} and R_{d} as defined above, and preferably chosen among OH, SH and NH₂.

According to a particular embodiment, the fluorine atom and the NH₂ group of the compound of formula (I) are present on the same side of the cyclopropane ring of compound (I).

According to another particular embodiment, the fluorine atom and the NH₂ group of the compound of formula (I) are present on the opposite side of the cyclopropane ring of compound (I).

The compound of the invention can be chosen in particular among:

| | | | |
|---|---|---|---|
| *cis*-I-1 | 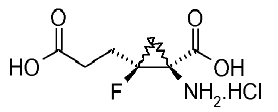 | *cis*-I-2 | 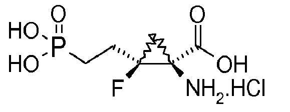 |
| *trans*-I-2 | 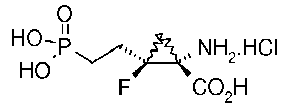 | *cis*-I-3 | 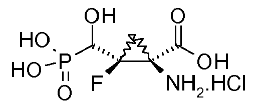 |
| *trans*-I-3 | 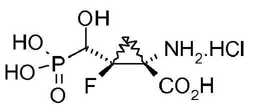 | *cis*-I-4 | 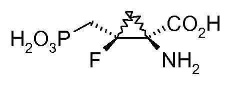 |
| *cis*-I-4a | 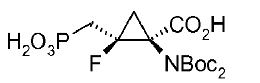 | *cis*-I-4b | 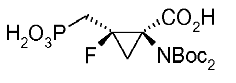 |
| *cis*-I-5 | 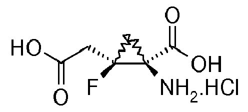 | *trans*-I-5 | 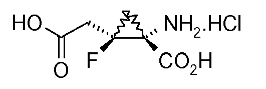 |
| *cis*-I-6 | 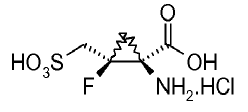 | *trans*-I-6 | 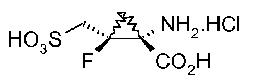 |

The present invention has for second object a compound of formula (I) as defined above, for its use as medicament, in particular in the treatment of neurological diseases, such as Alzheimer's disease, Parkinson's disease or epilepsy.

According to a particular embodiment of the present invention, R can be a (C₁-C₆)alkanediyl group, such as -CH₂- or -CH₂-CH₂-, substituted by a group chosen among PO₃H₂, CO₂H and SO₃H, and optionally substituted by one or more groups chosen among an halogen atom, ORₐ, SR_{b} and NR_{c}R_{d}, with Rₐ, R_{b}, R_{c} and R_{d} as defined above, and preferably chosen among OH, SH and NH₂.

According to a particular embodiment, the fluorine atom and the NH₂ group of the compound of formula (I) are present on the same side of the cyclopropane ring of compound (I).

According to another particular embodiment, the fluorine atom and the NH₂ group of the compound of formula (I) are present on the opposite side of the cyclopropane ring of compound (I).

In particular, the compounds of formula (I) can be chosen among:

| | | | |
|---|---|---|---|
| *cis*-I-1 | 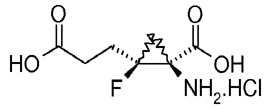 | *cis*-I-2 | 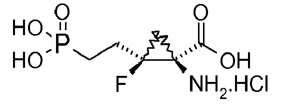 |
| *trans*-I-2 | 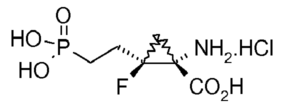 | *cis*-I-3 | 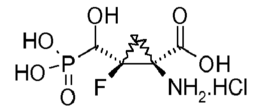 |
| *trans*-I-3 | 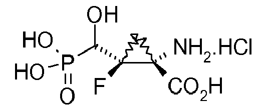 | *cis*-I-4 | 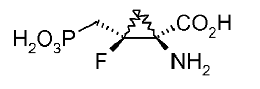 |
| *cis*-I-4a | 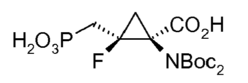 | *cis*-I-4b | 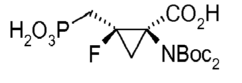 |
| *cis*-I-5 | 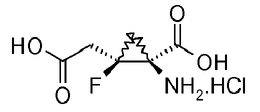 | *trans*-I-5 | 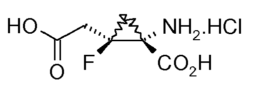 |
| *cis*-I-6 | 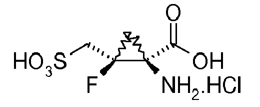 | *trans*-I-6 | 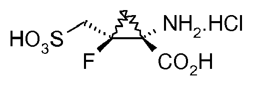 |

The present invention concerns also the use of a compound of formula (I) as defined above for the manufacture of a medicament, notably intended for the treatment of neurological diseases, such as Alzheimer's disease, Parkinson's disease or epilepsy.

The present invention concerns also a method for treating a neurological disease, such as Alzheimer's disease, Parkinson's disease or epilepsy, by administering an efficient amount of a compound of formula (I) as defined above to a patient in need thereof.

The present invention has for third object a pharmaceutical composition comprising at least one compound of formula (I) as defined above and at least one pharmaceutically acceptable excipient.

The pharmaceutical compositions of the invention can be intended to oral, sublingual, subcutaneous, intramuscular, intravenous, transdermal, local or rectal administration. The active ingredient can be administered in unit forms for administration, mixed with conventional pharmaceutical carriers, to animals or to humans. Suitable unit forms for administration comprise the forms for oral administration, such as tablets, gelatin capsules, powders, granules and oral solutions or suspensions, the forms for sublingual and buccal administration, the forms for subcutaneous, intramuscular, intravenous, intranasal or intraoccular administration and the forms for rectal administration.

When a solid composition is prepared in the form of tablets, the main active ingredient is mixed with a pharmaceutical vehicle such as gelatin, starch, lactose, magnesium stearate, talc, gum arabic and the like. The tablets may be coated with sucrose or with other suitable materials, or they may be treated in such a way that they have a prolonged or delayed activity and they continuously release a predetermined amount of active principle.

A preparation in gelatin capsules is obtained by mixing the active ingredient with a diluent and pouring the mixture obtained into soft or hard gelatin capsules.

A preparation in the form of a syrup or an elixir may contain the active ingredient together with a sweetener, an antiseptic, or also a taste enhancer or a suitable coloring agent.

The water-dispersible powders or granules may contain the active ingredient mixed with dispersing agents or wetting agents, or suspending agents, and with flavor correctors or sweeteners.

For rectal administration, suppositories are used which are prepared with binders which melt at rectal temperature, for example cocoa butter or polyethylene glycols.

For parenteral, intranasal or intraoccular administration, aqueous suspensions, isotonic saline solutions or sterile and injectable solutions which contain pharmacologically compatible dispersing agents and/or wetting agents are used.

The active principle may also be formulated in the form of microcapsules, optionally with one or more carrier additives.

The compounds of the invention can be used in a pharmaceutical composition at a dose ranging from 0.01 mg to 1000 mg a day, administered in only one dose once a day or in several doses along the day, for example twice a day. The daily administered dose is advantageously comprises between 5 mg and 500 mg, and more advantageously between 10 mg and 200 mg. However, it can be necessary to use doses out of these ranges, which could be noticed by the person skilled in the art

The pharmaceutical composition of the invention can further comprise another active compound, useful in particular in the treatment of neurological diseases, such as acetylcholinesterase inhibitors like donezepil, galanthamine, rivastigmine, mémantine or tacrine ; monoamine oxidase inhibitors like selegiline ; catecholamin-O-methyltransferase inhibitors like entacapone ; glutamatergic inhibitors like amantadine or baclofene ; cholinergic agonists like sabcomeline ; dopaminergic agonists like pergolide, cabergoline, ropirinole or pramipexole ; neuromediator analogs or precursors like L-3,4-dihydroxyphenylalanine ; and anticholinergics like trihexyphenidyl or tropatepine.

The present invention has for fourth object a pharmaceutical composition comprising:
(i) at least one compound of formula (I) as defined above, and
(ii) at least another active compound,
as a combination product for a simultaneous or separate use, or for use spread out over time.

The active compound can be useful in particular in the treatment of neurological diseases, and is advantageously chosen among acetylcholinesterase inhibitors like donezepil, galanthamine, rivastigmine, mémantine or tacrine ; monoamine oxidase inhibitors like selegiline ; catecholamin-O-methyltransferase inhibitors like entacapone ; glutamatergic inhibitors like amantadine or baclofene ; cholinergic agonists like sabcomeline ; dopaminergic agonists like pergolide, cabergoline, ropirinole or pramipexole ; neuromediator analogs or precursors like L-3,4-dihydroxyphenylalanine; and anticholinergics like trihexyphenidyl or tropatepine.

The present invention has for fifth object a pharmaceutical composition as defined above, according to the third and fourth object of the invention, for its use as medicament, in particular in the treatment of neurological diseases, such as Alzheimer's disease, Parkinson's disease or epilepsy.

The present invention has for sixth object a process for preparing a compound of formula (Ib) corresponding to a compound of formula (I) as defined above, in which R represents -CH₂CH₂R1 with R1 representing a direct bound or a (C₁-C₄)alkanediyl, optionally substituted by one or more groups chosen among an halogen atom, such as a fluorine atom, ORₐ, SR_{b}, NR_{c}R_{d}, PO(ORₑ)(OR_{f}), CO₂R_{g}, SO₂Rₕ, SO₃Rᵢ, PO(OH)(CH(OH)Rₖ), CN, N₃ or NH-C(=NH)NH₂,
with Rₐ, R_{b}, R_{c}, R_{d}, Rₑ, R_{f}, R_{g}, Rₕ, Rᵢ and Rₖ as defined above,
comprising the following successive steps:
(a) reaction of Horner-Wadsworth-Emmons between a compound of formula (IIa): 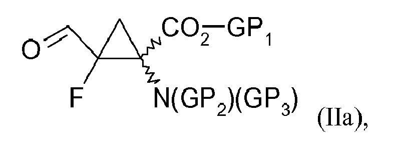 wherein GP₁ represents a O-protecting group, and GP₂ and GP₃ represent, independently of each other, a N-protecting group,
   and a compound of formula (III): 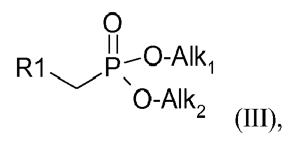 wherein Alk₁ and Alk₂ represent, independently of one another, a (C₁-C₆)alkyl group, such as an ethyl group, and R1 is as defined above,
   to give a compound of formula (IV): 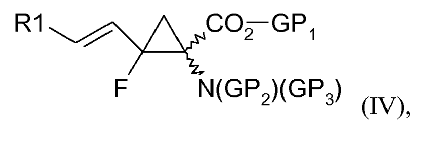 wherein R1, GP₁, GP₂ and GP₃ are as defined above,
(b) hydrogenation of the compound of formula (IV) obtained in the previous step (a) to give a compound of formula (V): 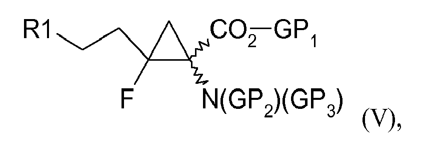 wherein R1, GP₁, GP₂ and GP₃ are as defined above,
(c) hydrolysis of the CO₂-GP₁ and N(GP₂)(GP₃) groups of the compound of formula (V) obtained in the previous step (b) to give a compound of formula (Ib): 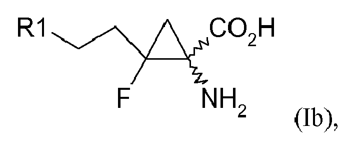 wherein R1 is as defined above, and
(d) separation of the compound (Ib) from the reaction mixture.

### Step a:

The term "O-Protecting group" as used in the present invention refers to a substituent which protects hydroxyl groups of a carboxylic acid function against undesirable reactions during synthetic procedures such as those O-protecting groups disclosed in Greene, "Protective Groups In Organic synthesis", (John Wiley & Sons, New York (1981)). O-protecting groups comprise (C₁-C₆)alkyl groups, such as methyl, ethyl *tert*-butyl; substituted methyl ethers, for example, methoxymethyl (MOM), benzyloxymethyl, 2-methoxyethoxymethyl, 2-(trimethylsilyl) ethoxymethyl and benzyl; and esters prepared by reacting the hydroxyl group with a carboxylic acid for example, acetate, propionate, benzoate and the like. Preferably, it will be a (C₁-C₆)alkyl group, and in particular a methyl or ethyl group.

The term "N-protecting group" as used in the present invention refers to those groups intended to protect an amino group against undesirable reactions during synthetic procedures. Commonly used N-protecting groups are disclosed in Greene, "Protective Groups In Organic Synthesis," (John Wiley & Sons, New York (1981)). N-protecting groups comprise carbamates, amides, N-alkyl derivatives, amino acetal derivatives, N-benzyl derivatives, imine derivatives, enamine derivatives and N-heteroatom derivatives. In particular, N-protecting groups include formyl, acetyl, trifluoroacetyl, benzoyl, pivaloyl, phenylsulfonyl, benzyl, t-butyloxycarbonyl (Boc), benzyloxycarbonyl (Cbz), trichloroethoxycarbonyl (TROC), 9-fluoroenylmethoxycarbonyl (Fmoc), allyloxycarbonyl (Alloc), acetyl, phtalimide, succinimide and the like. Preferably, it will be a Boc group.

Thus, step (a) will be advantageously carried out with N(GP₂)(GP₃) = NBoc₂.

Moreover, GP₁ will be advantageously a (C₁-C₆)alkyl group, such as an ethyl group.

Such a reaction of Horner-Wadsworth-Emmons is well known by the person skilled in the art. It can be carried out in the presence of a base such as triethylamine, notably in a solvent such as tétrahydrofurane (THF), with lithium bromide.

### Step b:

This step of hydrogenation can be carried out by classical procedures well known of the person skilled in the art. In particular, this reaction can be performed under an hydrogen atmosphere in the presence of a catalyst, such as palladium on carbon, in a solvent such as THF.

### Step c:

The reactions of hydrolysis are well known of the person skilled in the art and depend on the nature of the GP₁, GP₂ and GP₃ groups. The order of these two hydrolysis reactions is not important.

Moreover, these reactions can be carried out simultaneously if the two groups can be hydrolyzed in the same conditions. Thus, when GP₁ = (C₁-C₆)alkyl and N(GP₂)(GP₃) = N(Boc)₂, an acid treatment allows to hydrolyze simultaneously the CO₂-GP₁ and N(GP₂)(GP₃) groups to give respectively CO₂H and NH₂. The acid used in this reaction can be acetic acid, hydrochloride acid or a mixture thereof, notably a 1:1 mixture.

### Step d:

This step can be carried out by methods well known of the person skilled in the art, such as by extraction, evaporation or precipitation and filtration.

If necessary, the compound obtained can be further purified by classical methods, as, for example, by crystallization if the compound is crystalline, by distillation, by column chromatography on silica gel or else by high performance liquid chromatography (HPLC).

Supplementary steps of protection and deprotection of sensitive groups can be necessary, the skilled person being able to identify such sensitive groups and the method for protecting and deprotecting them.

The compound of formula (IIa), used as starting material in the synthesis of compounds of formula (Ib), can be prepared according to the following successive steps:
(a1)reaction between a compound of formula (VI): 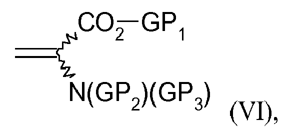 wherein GP₁, GP₂ and GP₃ are as defined above,
   and a compound of formula (VII),

   Br₂FC-C(O)Z-Alk₅ (VII),

   wherein Z represents O or NR2 with R2 representing an hydrogen atom, a (C₁-C₆)alkyl group or a (C₁-C₆)alkoxy group, and Alk₅ represents a (C₁-C₆)alkyl group, such as an ethyl group,
   in the presence of ZnY¹Y² with Y¹ representing a (C₁-C₆)alkyl group and Y² representing a bromine or iodine group or a (C₁-C₆)alkyl group to give a compound of formula (IIb): 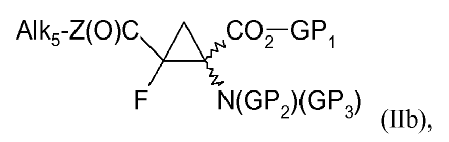 wherein GP₁, GP₂, GP₃, Z and Alk₅ are as defined above,
(b1)hydrolysis of the C(O)Z-Alk₅ group of the compound of formula (IIb) obtained in the previous step (a1) to give a compound of formula (IIc): 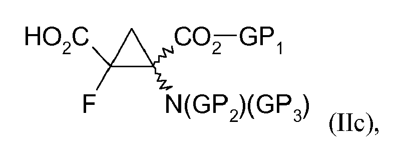 wherein GP₁, GP₂ and GP₃ are as defined above, and
(c1) reduction in aldehyde of the free carboxylic acid function of the compound of formula (IIc) obtained in the previous step (b1) to give a compound of formula (IIa).

### Step a1:

Advantageously, N(GP₂)(GP₃) represents NBoc₂, and GP₁ will represents preferably a (C₁-C₆)alkyl group.

In particular, Z can be an oxygen atom and ZnY¹Y² can be ZnEt₂, ZnMe₂, EtZnBr or EtZnI, and preferably is ZnEt₂.

### Step b1:

This hydrolysis reaction can be performed by basic treatment (saponification reaction), notably with a hydroxide of an alkaline metal such as LiOH, especially when Z = O.

### Step c1:

The reduction can be carried out by classical reduction methods of carboxylic acids in aldehydes, notably by treatment with diisobutyl aluminium hydride (DIBAL-H).

It is also possible to reduce the carboxylic acid derivative in the corresponding alcohol, notably by treatment with BH₃, and to further oxidize this alcohol in aldehyde, by the use of classical oxidizing agents for such a reaction like 2-iodoxybenzoic acid (IBX).

The present invention has for seventh object a compound of the following formula (II): 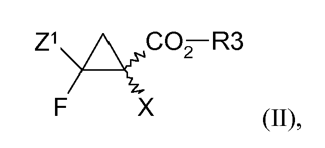 wherein:
- R3 represents an hydrogen atom or a GP₁ group as defined above,
- Z¹ represents a CHO, COOH, C(O)Z-Alk₅ or R group, with R, Z and Alk₅ as defined above, and
- X represents an NH₂, NHGP₂ or N(GP₂)(GP₃) group, with GP₂ and GP₃ as defined above,
provided that R3 does not represent an hydrogen atom when X represents a NH₂ group.

Such compounds are particularly useful as synthesis intermediates in the preparation of the compounds of formula (I). Indeed, the compounds of formula (I) can be prepared by classical coupling reaction from these intermediates, and in particular from compounds of formula (IIa), (IIb), (IIc), (IId) and (IIe), optionally followed by classical hydrolysis reactions to obtain the free carboxylic acid and amino groups of the cyclopropane moiety. The coupling reaction can be a Wittig or an Horner-Wadsworth-Emmons reaction as shown previously, or else a Mitsunobu reaction, an Arbuzov reaction, and the like.

Moreover, when R3 = H or X = NH₂, these compounds can be used in a peptide synthesis, by peptide coupling from the free carboxylic acid or amine group, the protected carboxylic acid or amine group being further deprotected in order to be engaged in a new peptide coupling to pursue the peptide synthesis.

According to a first aspect, Z¹ represents a radical R as defined above and in particular a (C₁-C₆)alkanediyl group, such as -CH₂- or -CH₂-CH₂-, substituted by a group chosen among PO₃H₂, CO₂H and SO₃H, and optionally substituted by one or more groups chosen among an halogen atom, ORₐ, SR_{b} and NR_{c}R_{d}, with Rₐ, R_{b}, R_{c} and R_{d} as defined above. This kind of compounds corresponds thus to advanced synthesis intermediates in the preparation of the compounds of formula (I).

Moreover, X will represent advantageously a N(GP₂)(GP₃) group and in particular a NBoc₂ group, and R3 will be advantageously a GP₁ group such as a (C₁-C₆)alkyl group.

According to a second aspect, the compound is a compound of formula (II) in which R3 = H or X = NH₂. As indicated above, this kind of compounds is useful in a peptide synthesis as new synthetic amino acids. In this case, Z¹ will be advantageously a radical R as defined above.

According to a third aspect, Z¹ represents a CHO, COOH or C(O)Z-Alk₅ group as defined above. This kind of compounds corresponds thus to early synthesis intermediates in the preparation of the compounds of formula (I).

In this case, X will represent advantageously a N(GP₂)(GP₃) group and in particular a NBoc₂ group, and R3 will be advantageously a GP₁ group such as a (C₁-C₆)alkyl group.

A compound of formula (II) can be chosen in particular among:

| | | | |
|---|---|---|---|
| *cis*-II-1 | 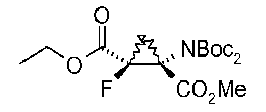 | *trans*-II-1 | 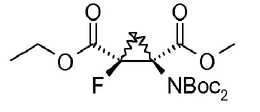 |
| II-2 | 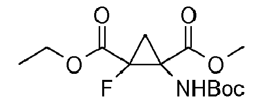 | II-3 | 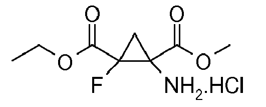 |
| *cis*-II-4 | 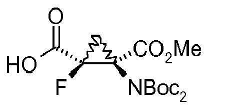 | *trans*-II-4 | 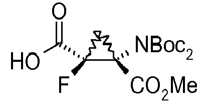 |
| *cis*-II-4a | 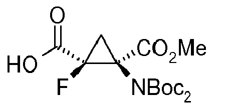 | *cis*-II-4b | 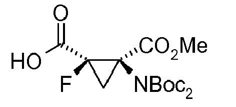 |
| *cis*-II-5 | 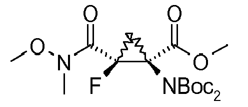 | *trans*-II-5 | 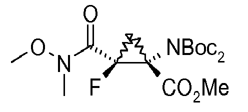 |
| *cis*-II-6 | 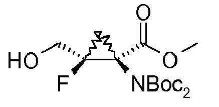 | *trans*-II-6 | 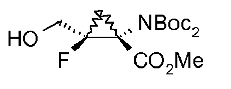 |
| *cis*-II-7 | 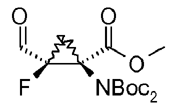 | *trans*-II-7 | 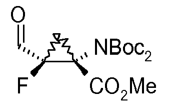 |
| *cis*-II-8 | 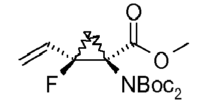 | *cis*-II-9 | 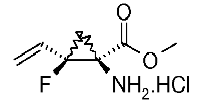 |
| *cis*-II-10 | 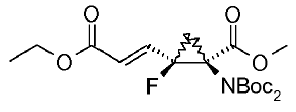 | *trans-*II-10 | 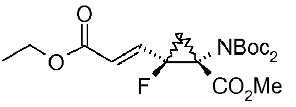 |
| *cis*-II-11 | 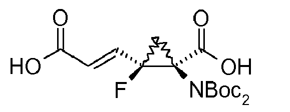 | *cis*-II-12 | 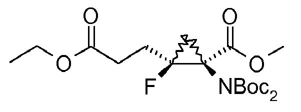 |
| *trans*-II-12 | 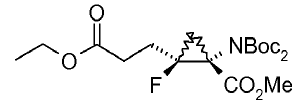 | *cis*-II-13 | 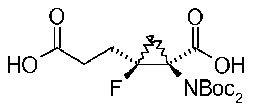 |
| *cis*-II-14 | 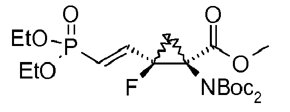 | *trans*-II-14 | 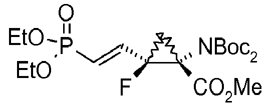 |
| *cis*-II-15 | 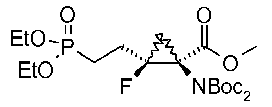 | *trans-*II-15 | 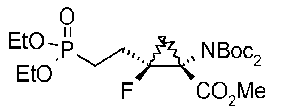 |
| *cis*-II-16 | 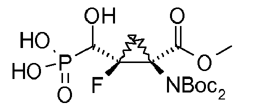 | *trans*-II-16 | 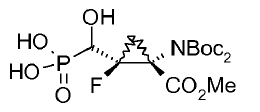 |
| II-17 | 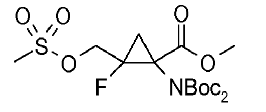 | II-18 | 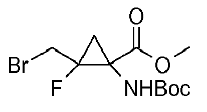 |
| II-19 | 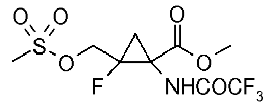 | II-20 | 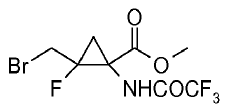 |
| II-21 | 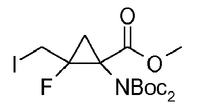 | *cis*-II-22 | 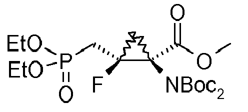 |
| *cis*-II-23 | 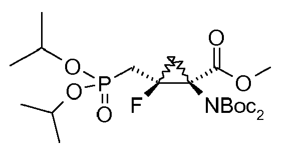 | *cis*-II-24 | 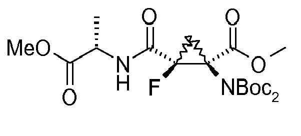 |
| *trans*-II-24 | 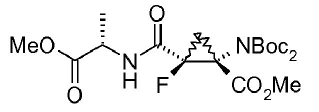 | *cis*-II-25 | 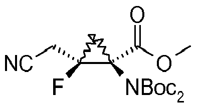 |
| *cis*-II-26 | 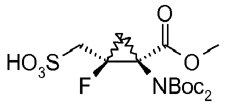 | *trans*-II-26 | 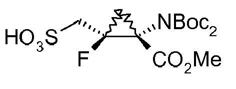 |
| *cis*-II-27 | 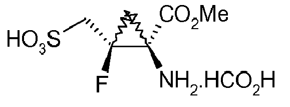 | *trans*-II-27 | 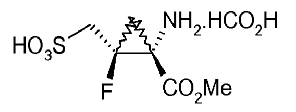 |
| *trans*-II-28 | 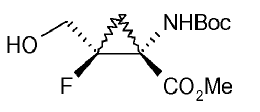 | *trans*-II-29 | 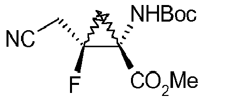 |

The present invention has for eighth object a process for preparing a compound of formula (II) as defined above in which Z¹ represents a CHO, COOH, C(O)Z-Alk₅, CH₂OH or CH₂Hal group, R3 represents an hydrogen atom and X represents a N(GP₂)(GP₃) group as defined above, with Alk₅ as defined above and Hal representing an halogen atom, comprising the following successive steps:
(a2) reaction between a compound of formula (VI) as defined above,
   and a compound of formula (VII) as defined above,
   in the presence of ZnY¹Y² with Y¹ and Y² as defined above to give a
   compound of formula (IIb) as defined above,
(b2)optionally hydrolysis of the the C(O)Z-Alk₅ group of the compound of formula (IIb) obtained in the previous step (a2) to give a compound of formula (IIc) as defined above,
(c2) optionally reduction in aldehyde or alcohol of the free carboxylic function of the compound of formula (IIc) obtained in the previous step (b2) to give a compound of formula (IIa) as defined above or a compound of the following formula (IId), 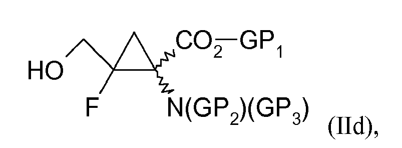 wherein GP₁, GP₂ and GP₃ are as defined above,
(d2)optionally halogenation of the alcohol moiety of the compound of formula (IId) obtained at the previous step (c2) to give a compound of the following formula (IIe), 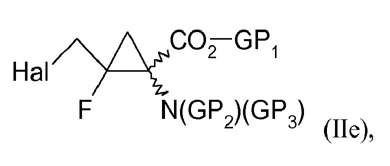 wherein Hal, GP₁, GP₂ and GP₃ are as defined above, and
(e2) separation of the compound of formula (IIa), (IIb), (IIc), (IId) or (IIe) from the reaction mixture.

### Steps a2 and b2:

Steps (a2) and (b2) are identical to the respective steps (a1) and (b1). Consequently, the remarks made previously for steps (a1) and (b1) apply also for steps (a2) and (b2) respectively.

### Step c2:

The reduction of the carboxylic acid in alcohol or in aldehyde can be carried out by classical reduction methods, notably by treatment with diisobutyl aluminium hydride (DIBAL-H) to obtain an aldehyde or by treatment with BH₃ to obtain an alcohol.

It is also possible to obtain the aldehyde derivative by oxidation of the alcohol by using classical oxidizing agents for such a reaction like 2-iodoxybenzoic acid (IBX).

### Step d2:

This step can be carried out by direct halogenation of the alcohol derivative by using well known halogenating agent or preferably by converting the alcohol moiety of the compound of formula (IId) into a leaving group (such as a methanesulfonyloxy group) and by further carrying out a nucleophile substitution with an halogenated derivative such as an halide of an alkali metal (for example LiBr or NaI).

### Step e2:

This step can be carried out by methods well known of the person skilled in the art, such as by extraction, evaporation or precipitation and filtration.

If necessary, the compound obtained can be further purified by classical methods, as, for example, by crystallization if the compound is crystalline, by distillation, by column chromatography on silica gel or else by high performance liquid chromatography (HPLC).

Supplementary steps of protection and deprotection can be necessary, the skilled person being able to identify such methods when there are necessary.

### EXEMPLES

### 1. Chemical synthesis of the compounds of the invention

### 1.1. General information

- Experiments involving organometallics were carried out under argon atmosphere. All moisture-sensitive reactants were handled under argon atmosphere.
- Low temperature experiments were carried out by cooling down the flasks with an acetone bath frozen by dry-ice. The flasks were equipped with septum caps.
- All the compounds have been prepared as a racemic mixture except otherwise stated.

### 1.2. Synthesis of compounds of formula (II)

### ►Boc-Ser-OMe (1)

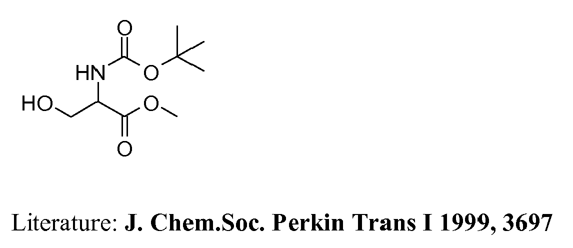

To a suspension of serine methyl ester hydrochloride (4.4 g, 28 mmol, 1 equiv.) in dichloromethane (20mL) cooled at 0°C, triethylamine (7.73 mL, 61.6 mmol, 2.2 equiv.) was added followed by Boc₂O (6.86 g, 30.8 mmol, 1.1 equiv.) previously solubilised in dichloromethane (20 mL). The mixture was allowed to warm to room temperature and stirred overnight. Solvents were removed under vacuo, the oily crude product was taken up in ethyl acetate (25 mL), washed successively with KHSO₄ 1N (2x20 mL), NaHCO₃ 1N (20 mL), brine (20 mL), dried over MgSO₄ and concentrated under vacuo to afford 6.0 g of desired product (98% yield, yellow oil) which was used without further purification.

The obtained ¹H NMR and ¹³C NMR spectra comply with the structure of the compound.

**IR** (film) : 3390, 2978, 1744, 1697, 1514, 1368, 1165 cm⁻¹

### ►Boc-ΔAla(N-Boc)-OMe (2)

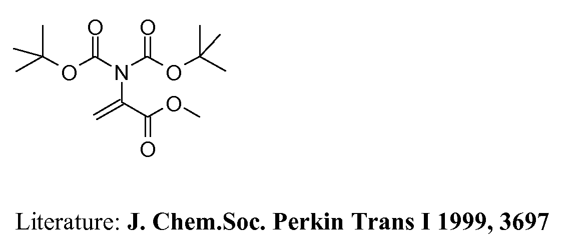

To a solution of Boc-Ser-OMe 1 (36.6 g, 167 mmol, 1 equiv.) in acetonitrile (160 mL) cooled at 0°C, DMAP (4.08 g, 33.4 mmol, 0.2 equiv.) was added followed by Boc₂O portionwise (82.7 g, 367 mmol, 2 equiv.). The mixture was allowed to stir at room temperature for 15 minutes and was then heated at 60°C until starting material was totally consummed (TLC monitoring). Solvents were removed under vacuo, the resulting oily crude product was taken up in ethyl acetate (100 mL), washed successively with KHSO₄ 1N (2x100 mL), NaHCO₃ 1N (100 mL), brine (100 mL), dried over MgSO₄ and concentrated under vacuo. The resulting oil was storred at -20°C overnight to afford 48.8 g of desired product (97% yield, white solid) as a white solid which was used without further purification.

Note: On large scale synthesis, first portions of Boc₂O have to be added very slowly on DMAP because of large quantities of carbon dioxide formed.

The obtained ¹H NMR and ¹³C NMR spectra comply with the structure of the compound.

**IR** (film) : 1795, 1755, 1732 cm⁻¹

| | | |
|---|---|---|
| **Elemental Analysis** | Calculated | C 55.80 % ; H 7.69 % ; N 4.65 % |
| | Found | C 55.87 % ; H 7.47 % ; N 4.68 % |

### ►Methyl 1-(N,N-diterbutyloxycarbonyl)amino-2-fluoro-2-methoxycarbonyl cyclopropylcarboxylate (II-1)

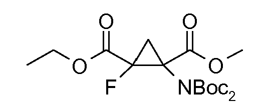

A solution under nitrogen of Boc-ΔAla(*N*-Boc)-OMe 2 (20.5 g, 68.2 mmol, 1 equiv.) and dibromofluoroethyl acetate (19.6 mL, 136.4 mmol, 2 equiv.) in dry THF (60 mL) was heated at 50°C. Diethyl zinc 1N in hexanes (137 mL, 137 mmol, 2 equiv.) was added dropwise over 2 hours *via* a syringe pump and the resulting mixture was stirred 3 h at this temperature. After cooling at room temperature, the mixture was poured into a stirred mixture of water (150 mL) and diethylether (150 mL). The heterogeneous mixture was filtered through a pad of celite, organic layer was separated and aqueous layer was extracted with diethylether. Organic layers were assembled, washed with 100 mL of brine and dried over MgSO₄. Removal of solvent afford 33 g of crude product which was purified by column chromatography (5% ethyl acetate, 1% triethylamine in cyclohexane) to afford 17.4 g (63 %, yellow oil) of pure desired product as a mixture of *cis* and *trans* diastereoisomeres (67:33).

The obtained ¹H NMR, ¹³C NMR and ¹⁹F NMR spectra comply with the structure of *cis* and *trans* diastereoisomeres.

**MS** (ESI/positive mode) : *m*/*z* (relative abundance) ; 428.20 [M+Na]⁺ (45), 833.20 [2M+Na]⁺ (100)

**IR** (film) : 3445, 1799, 1748, 1371, 1278, 1255, 1159, 1122, 1101, 1027, 855, 785 cm⁻¹

| | | |
|---|---|---|
| **Elemental Analysis** | Calculated | C 53.33 % ; H 6.96 % ; N 3.45 % |
| | Found | C 52.95 %; H 6.64 %; N 3.71 % |

### ►Methyl 1-(N-terbutyloxycarbonyl)amino-2-ethyloxycarbonylcyclopropyl-carboxylate (II-2)

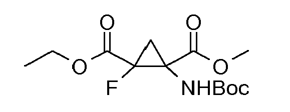

Literature: J. Org. Chem. 1988, 53, 3843 **I1-1** (824 mg, 2.0 mmol, 1 equiv.) was solubilised under argon in dry dichloromethane (2 mL). Trifluoroacetic acid (226 µL, 3.0 mmol, 1.5 equiv.) was added and the mixture was stirred 22 h at room temperature. The mixture was then poured into Et₂O (60 mL), washed successively with 10 % aqueous sodium hydroxyde (10 mL), brine (10 mL), and dried over MgSO₄. Removal of solvent affords 610 mg (98 %, yellow oil) of pure desired product as a mixture of cis and trans diastereoisomere (67:33).

The obtained ¹H NMR, ¹³C NMR and ¹⁹F NMR spectra comply with the structure of *cis* and *trans* diastereoisomeres.

**MS** (CI/positive mode) : *m*/*z* (relative abundance) ; 206 (20), 250 (100), 306 [M+H⁺] (38)

**IR (film) :** 3366, 746, 1504, 1440, 1371, 1282, 1253, 1218, 1163, 1095, 915, 860, 778, 734

### ► Mehyl 1-amino-2-ethyloxycarbonylcyclopropyl-carboxylate (II-3)

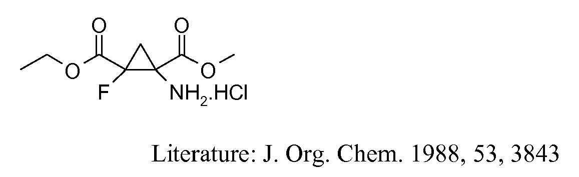

### From product II-2

To a solution of **II-2** (600 mg, 1.96 mmol, 1 equiv.) in methanol (13 mL) cooled at 0°C, was added a 5-6 M HCl solution in isopropanol (3.6 mL, -10 equiv.). The mixture was stirred 5 h at this temperature. Removal of solvent affort an oily crude product which was taken up in Et₂O (5 mL). The resulting precipitated solid was filtered and washed with Et₂O (2x2 mL) to furnish 331 mg (70 %) of desired product as a mixture of *cis* and *trans* isomer (76:24).

### From product II-1

**II-1** (147 mg, 0.36 mmol, 1 equiv.) was solubilised in HCl saturated EtOAc solution (5 mL) cooled at 0 °C. The mixture was stirred 2 hours at this temperature. Solvent was removed, the resulting oily crude product was taken up in Et₂O (5 mL). The resulting precipitated solid was filtered and washed with Et₂O (2x2 mL) to furnish 49 mg (57 %, white solid) of desired product as a mixture of *cis* and *trans* isomer (82:18).

The obtained ¹H NMR, ¹³C NMR and ¹⁹F NMR spectra comply with the structure of *cis* and *trans* diastereoisomeres.

**MS** (ESI/positive mode) : *m*/*z* (relative abundance) ; 185.93 (35), 205.93 [M+H]⁺ (100)

**IR** (KBr) : 3138, 3008, 2674, 1766, 1544, 1407, 1279, 1196, 1172, 1023, 968, 747 cm⁻¹

| | | |
|---|---|---|
| **Elemental Analysis** | Calculated | C 39.76 % ; H 5.42 % ; N 5.80 % |
| | Found | C 39.64 % ; H 5.76 % ; N 5.76 % |

### ► 2-(N,N-di-tert-butoxycarbonyl)amino-1-fluoro-2-methoxycarbonyl cyclopropyl methanoic acid with a cis configuration (cis-II-4)

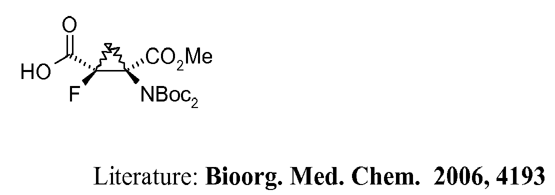

**II-1** (19.83 g, 48.9 mmol, 1 equiv.) was solubilised in a solution of THF:water 10:1 (300 mL) and was cooled at 0°C. A molar solution of lithium hydroxyde (73 mL, 73 mmol, 1.5 equiv.) was added dropwise and the mixture was stirred at this temperature for 2 hours. The solution was then acidified to pH = 2 by addition of a solution of HCl 1N and extracted with EtOAc (3x200 mL). Sovents were removed and the oily mixture was taken up in Et₂O. Desired acid ***cis*-II-4** in its sodium salt form was extracted with Na₂CO₃ 0.5M (3x100 mL), the organic layer was washed with brine, dried over MgSO₄ and concentrated under vacuo to afford 7.05 g (36 %) of ***trans*-II-1.** The aqueous layer was re-acidified at 0°C by addition of HCl 4N to pH = 2, extracted with diethylether (4x100), dried over MgSO₄ and concentrated to furnish 11.43 g (62 %, yellow oil) of ***cis*-II-4.**

The obtained ¹H NMR, ¹³C NMR and ¹⁹F NMR spectra comply with the structure of the compound.

**MS** (ESI), positive mode : *mlz* 400.20 [M+Na]⁺; negative mode : *mlz* 376.73 [M-H]⁺

**IR** (film) : 3500, 2981, 2927, 1748, 1371, 1283, 1156, 1112, 852, 772 cm⁻¹

| | | |
|---|---|---|
| **Elemental Analysis** | Calculated | C 50.92 % ; H 6.41 % ; N 3.71 % |
| | Found | C 51.13 % ; H 6.65 % ; N 3.76 % |

A chiral resolution of racemate ***cis*-II-4** has been also carried out according to the standard procedure described below, in order to obtain ***cis*-II-4** batches enriched in only one of the two optical isomers **(*cis*-II-4a** and ***cis*-II-4b)** (or almost in a pure form) in a very reproducible manner.

Thus, the first crystallisation is based on the following quantities:
- 1.00 g of crystallised racemic ***cis*-II-4** (2.65 mmol)
- 0.438 g of enantiomerically pure (-)-ephedrine (2.65 mmol)
- if this crystallisation is carried out using (-)-ephedrine hemihydrate, the initial mass to be introduced is 0.462 g. Dissolution in a minimum of ethyl acetate followed by removal of solvent *in vacuo* (T=50°C, P#6-8mbar) enables elimination of water to obtain a dry solid.

Formation of (-)-ephedrine salts should lead to 1.438 g of solid. In order to allow the formation of only one crystallised salt, the quantity of ethyl acetate to be introduced must be 96.2% by weight of the global mixture:

| **Product** | **% by weight** | **Mass (g)** |
|---|---|---|
| (-)-ephedrine salt | 3.8 | 1.438 |
| Ethyl acetate | 96.2 | 36.400 |

The reactants are mixed together at room temperature. After few minutes of vigorous stirring (using a magnetic stirrer), the first solid particles are noticed. The mixture is let to stir in these conditions for at least 24 hours, and the crystals are filtered through a sintered glass funnel. The solid is then dried over filter paper and in a ventilated oven at 40°C for few hours. The yield of this crystallisation highly depends on the purity of the initial ***cis*-II-4** batch. In this particular example, a yield of 11.1% was obtained, corresponding to 160 mg of the resulting salt with an enantiomeric excess approaching 100%. The pure enantiomer **(**α**)-*cis*-II-4** is obtained after acid hydrolysis (described below).

In order to avoid loss of uncrystallised compound, the sintered funnel is washed with ethyl acetate. The liquid phases are combined with the mother liquor in a tared round-bottom-flask and the solvent is removed *in vacuo.* If no loss of compound occurred, the total mass of recovered solid must be close to 1.278 g. The procedure for ***cis*-II-4** release is then applied, affording a viscous solid. The enantiomeric excess of this phase can be measured via chromatography (HPLC or GC) in order to evaluate the proportion of each enantiomers. Generally, the measured ee of the product recovered from the liquid phase is comprised between 15% and 20%; in this particular example, 15% ee (i.e. the recovered mixture is composed of 42.5% by weight of **(**α**)-*cis*-II-4** and 57.5% by weight of **(**β**)-*cis*-II-4).**

To this solid, anhydrous (+)-ephedrine is added:
- 0.889 g of ***cis***-**II-4** with an enantiomeric excess comprised between 15% and 20%
- 0.389 g of (+)-ephedrine
- in the case of (+)-ephedrine hemihydrate, the mass to be introduced is 0.410 g, using the same procedure described hereabove.

Formation of (+)-ephedrine salts should lead to 1.278 g of solid. In order to allow the formation of only one crystallised salt, the quantity of ethyl acetate to be introduced must be 95.56% by weight of the global mixture:

| Product | % by weight | Mass (g) |
|---|---|---|
| (+)-ephedrine salts | 4.44 | 1.278 |
| Ethyl acetate | 95.56 | 27.506 |

Using the aforementioned procedure, the compound is crystallised in ethyl acetate over 24 hours and filtered through a sintered glass funnel. The obtained salt is enriched in **(**β**)-*cis***-**II**-**4** with an enantiomeric excess approaching 100%.

The mother liquor is treated as described hereabove, leading to a mixture of both enantiomers of ***cis*-II-4** with a 15% ee. Therefore, the same percent by weight proportions can be applied for the next crystallisations until the mixture is fully resolved, which is not common.

The procedure for the release of ***cis*-II-4** via acid hydrolysis of **cis-II-4** ephedrine salts is as follows:

***cis*-II-4** can be released either from a solid crystallised salt, or from a liquid phase (mother liquor). In the latter case, removal of the solvent *in vacuo* is necessary until obtaining a solid. In both cases, the free acid is released after addition of a 0.1 M solution of hydrochloric acid, using the proportions described in the following table.

| Product | Mass (g) | N / 10⁻³ mole | Volume |
|---|---|---|---|
| Salt(s) *^{(a)}* | 1 | 1.84 | |
| HCl (0.1M) | | 1.84 | 1.2 x 18.4 mL |
| Diethyl ether | | | 4 x 18.4 mL |

| | | | |
|---|---|---|---|
| *^{(a)}* molecular weight of the ephedrine salt: 540.60 g.mol⁻¹ | | | |

1.2 x 18.4 mL of 0.1 M HCl (i.e. 22.08 mL) are added to the ***cis*-II-4** ephedrine salt under vigorous magnetic stirring in order to release the free acid form of ***cis*-II-4.** A very viscous solid precipitates, which is dissolved and extracted via addition of diethyl ether (18.4 mL). The phases are separated, and the aqueous phase is extracted with a further 3 x 18.4 mL of Et₂O. The organic layers are combined, dried over MgSO₄ under stirring over a period of 30 minutes, filtered and evaporated *in vacuo* to afford ***cis*-II-4.**

### ► 2-(N,N-di-tert-butoxycarbonyl)amino-1-fluoro-2-methoxycarbonyl cyclopropylmethanoic acid with a trans configuration (trans-II-4)

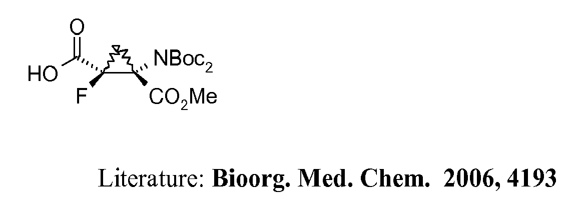

Diester ***trans*-II-1** (1.64 g, 4.04 mmol, 1 equiv.) was solubilised in a solution of THF:water 5:2 (70 mL) and was cooled at 0°C. A molar solution of lithium hydroxyde (4.45 mL, 4.45 mmol, 1,1 equiv.) was added dropwise and the mixture was allowed to warm to room temperature and stirred for 20 hours. The solution was then acidified by addition of a solution of HCl 1N to pH 3 and extracted with diethylether (200 mL). Organic layer was dried over MgSO₄ and concentrated to furnish 1.31 g (86 %, yellow oil) of ***trans*-II-4.**

The obtained ¹H NMR, ¹³C NMR and ¹⁹F NMR spectra comply with the structure of the compound.

**MS** (ESI, negative mode) : *m*/*z* 376.36 [M-H]⁻

**IR** (film) : 3438, 2981, 1747, 1371, 1289, 1158, 1108, 855, 772 cm⁻¹

### ► Methyl 1-(N,N-diterbutyloxycarbonyl)-2-(N,O-dimethylhydroxylamino carbonyl)-2-fluorocyclopropyl carboxylate with a cis configuration (cis-II-5)

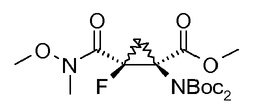

To a solution of ***cis*-II-4** (140 mg, 0.34 mmol, 1 equiv.) and *N,O-*dimethylhydroxylamine hydrochloride (53 mg, 0.54 mmol, 1.6 equiv.) in dry dichloromethane (1 mL), dicyclohexylcarbodiimide (72 mg, 0.34 mmol, 1 equiv.) was added. The mixture was stirred at room temperature for 10 minutes and triethylamine (192 µL, 1.38 mmol, 4 equiv.) was added. Consumption of starting material was controlled by ¹⁹F NMR. Solvants were removed under vacuo, crude mixture was taken up in acetone, filtered and concentrated. The oily residue was dissolved in dichloromethane (10 mL), washed successively with sodium carbonate 1N (10 mL) and brine (10 mL). Organic layer was dried over magnesium sulfate and concentrated under reduced pressure to afford 90 mg (62 %, yellow oil) of desired product ***cis*-II-5.**

The obtained ¹H NMR, ¹³C NMR and ¹⁹F NMR spectra comply with the structure of the compound.

**MS** (ESI/mode positive) : *m*/*z* (abondance relative) ; 264.93 (7), 343.00 (12), 443.07 [M+Na]⁺ (100)

**IR** (film) : 3445, 1799, 1748, 1371, 1278, 1255, 1223, 1159, 1101, 1027, 855, 785 cm⁻¹

### ► Methyl 1-(N,N-diterbutyloxycarbonyl)-2-(N,O-dimethylhydroxylamino carbonyl)-2-fluorocyclopropyl carboxylate with a trans configuration (trans-II-5)

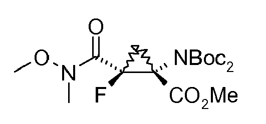

Compound ***trans*-II-4** (54 mg, 0.14 mmol, 1 equiv.), alanine methyl ester hydrochloride (31 mg, 0.16 mmol, 1.1 equiv.), HOBT (21 mg, 0.15 mmol, 1.05 equiv.) and *N*-methylmorpholine (48 µL, 0.44 mmol, 3.05 equiv.) were solubilised in dry dichloromethane (5 mL). EDCI (29 mg, 0.15 mmol, 1.05 equiv.) was added and the mixture was stirred at room temperature for 36 hours. The reaction was quenched with water (10 mL), extracted with dichloromethane (3 x 10 mL), organic layer was washed with brine (10 mL), dried over MgSO₄ and concentrated under reduced pressure. The oily crude product was purified on silica gel (10% EtOAc in cyclohexane) to furnish 51 mg (77 %, white solid) of *trans*-**140** as a mixture of diastéréoisomers (1:1).

The obtained ¹H NMR, ¹³C NMR and ¹⁹F NMR spectra comply with the structure of the compound.

**MS** (ESI/positive mode) : *m*/*z* (relative abundance) ; 443.07 [M+Na⁺] (39), 420.40 [M+H⁺] (20), 320.87 (38), 264.93 (100), 200.93 (26)

**IR** (film) : 3400, 2924, 1745, 1667, 1434, 1368, 1278, 1167, 1122, 1028, 863, 771 cm⁻¹

| | | |
|---|---|---|
| **Elemental Analysis** | Calculated | C 51.42 % ; H 6.95 % ; N 6.66 % |
| | Found | C 51.69 % ; H 6.98 % ; N 6.49 % |

### ► Methyl 1-(N,N-diterbutyloxycarbonyl)-2-(hydroxymethyl)-2-fluoro cyclopropyl carboxylate with a cis configuration (cis-II-6)

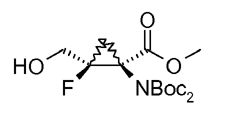

Carboxylic acid ***cis*-II-4** (16.0 g, 42.5 mmol, 1 equiv.) was dissolved at 0°C in anhydrous THF (250 mL) under argon. A 1M solution of BH₃.THF (130 mL, 130 mmol, 3 equiv.) was added dropwise over a period of 30 minutes. The reaction was allowed to warm to room temperature and stirred overnight. Upon completion (TLC and ¹⁹F NMR monitoring), it was then quenched by dropwise addition of water (150 mL), extracted with Et₂O (100 mL), diethylacetate (2x100 mL), washed with brine (100 mL) and dried over magnesium sulfate. Removal of solvant and purification by column chromatography (5% ethyl acetate, 1% triethylamine in cyclohexane) afforded 15.07g (97 %, yellow oil) of pure desired alcohol ***cis*-II-6.**

The obtained ¹H NMR, ¹³C NMR and ¹⁹F NMR spectra comply with the structure of the compound.

**MS** (ESI/positive mode) : *m*/*z* (relative abundance) ; 207.87 (83), 286.00 (22), 385.93 [M+Na]⁺ (92), 748.93 [2M+Na]⁺

**IR** (film) : 3436, 2924, 2854, 1736, 1456, 1370, 1281, 1157, 1121, 1042, 762 cm⁻¹

### ► trans-Methyl 1-(N,N-diterbutyloxycarbonyl)-2-(hydroxymethyl)-2-fluoro cyclopropyl carboxylate with a trans configuration (trans-II-6)

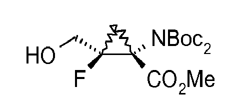

Carboxylic acid ***trans*-II-4** (3.40 g, 9.0 mmol, 1 equiv.) was solubilised in anhydrous THF (30 mL) under argon. A 1M solution of BH₃.THF complex (63 mL, 63 mmol, 7 equiv.) was added dropwise over a period of 20 minutes. The reaction was allowed to warm to room temperature and stirred overnight. Upon completion (TLC and ¹⁹F NMR monitoring), it was then quenched with 2M HCl (20 mL). Water was added (50 mL), and the mixture was extracted with Et₂O. Organic phases were combined, washed with NH₄Cl, water, brine, dried over magnesium sulfate and concentrated. The resulting oily crude oil was purified by column chromatography (9% ethyl acetate, 1% triethylamine in cyclohexane) to afford 3.16 g (97 %, colorless oil) of pure desired alcohol.

The obtained ¹H NMR, ¹³C NMR and ¹⁹F NMR spectra comply with the structure of the compound.

**MS** (ESI/positive mode) : *m*/*z* (relative abundance) ; 363.80 [M+H]⁺ (34), 381.00 [M+H₃0]⁺ (48), 386.07 [M+Na]⁺ (36), 743.60 [2M+H₃O]⁺ (100), 748.93 [2M+Na]⁺

**IR** (film) : 3418, 2930, 2359, 1746, 1435, 1370, 1248, 1157, 1123, 1046, 849, 771 cm⁻¹

### ► Methyl 1-(N,N-diterbutyloxycarbonyl)-2-(oxomethyl)-2-fluoro cyclopropyl carboxylate with a cis configuration (cis-II-7)

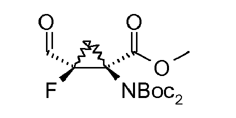

### Reductive procedure

To a cooled solution (-78 °C) of ***cis*-II-5** (150 mg, 0.35 mmol, 1 equiv.) in dry THF (2.5 mL), was added dropwise at -78 °C a solution of diisobutyl aluminium hydride (DIBAL-H) 1N in dichloromethane (0.53 mL, 0.53 mmol, 1.5 equiv.). The resulting mixture was stirred at the same temperature for 3 hours and was quenched by addition of saturated aqueous ammonium chloride (1 mL). After warming to room temperature, HCl 1N (2 mL) was added, the organic layer was extracted with diethyl ether (3x5 mL) and washed successively with saturated aqueous sodium hydrogenocarbonate (2 mL) and brine (2 mL). Organic layer was dried over MgSO₄ and removal of solvent afforded an oily crude product which was purified by column chromatography (10% ethyl acetate in cyclohexane) to furnish 60 mg (47 %, yellow oil) of pure desired aldehyde.

### Oxidative procedure

To a solution of ***cis*-II-6** (1 equiv.) in non-distillated EtOAc (0.5 M), IBX (3 equiv.) was added. The resulting suspension was heated under stirring at reflux. End of reaction was monitored by TLC. Solvent was removed, the resulting white heterogeneous oil was taken up in diethylether and filtered. The filtrate was concentrated under reduced pressure to afford quantitatively desired aldehyde which can either be used without further purification or purified by column chromatography (5% ethyl acetate in cyclohexane).

The obtained ¹H NMR, ¹³C NMR and ¹⁹F NMR spectra comply with the structure of the compound.

**MS** (ESI/positive mode) : *m*/*z* (relative abundance) ; 162.13 (10), 316.33 (6), 384.27 (11), 416.27 [M + MeOH + Na]⁺ (100), 609.80 (15), 745.33 [2M+Na]⁺

**IR** (film) : 3426, 1799, 1736, 1458, 1369, 1276, 1254, 1156, 1121, 829, 784 cm⁻¹

| | | |
|---|---|---|
| **Elemental Analysis** | Calculated | C 53.18 % ; H 6.69 % ; N 3.88 % |
| | Found | C 53.34 % ; H 6.78 % ; N 3.77 % |

### ► trans-Methyl 1-(N,N-diterbutyloxycarbonyl)-2-carbonyl-2-fluoro cyclopropyl carboxylate with a trans configuration (trans-II-7)

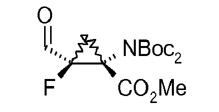

The same procedure as described above for preparing ***cis*-II-7** was applied to ***trans-*II-5** or ***trans*-II-6** to furnish compound ***trans*-II-7** as a yellow oil.

The obtained ¹H NMR, ¹³C NMR and ¹⁹F NMR spectra comply with the structure of the compound.

**IR** (film) : 3393, 2980, 1737, 1439, 1370, 1253, 1159, 1123, 850, 773 cm⁻¹

| | | |
|---|---|---|
| **Elemental Analysis** | Calculated | C 53.18 % ; H 6.69 % ; N 3.88 % |
| | Found | C 53.47 %; H 6.76 %; N 4.06 % |

### ► Methyl 1-(N,N-diterbutyloxycarbonyl)amino-2-vinyl-2-fluoro cyclopropyl carboxylate with a cis configuration (cis-II-8)

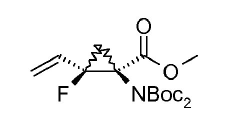

To a solution under argon and cooled at 0°C of methylphosphonium bromide (222 mg, 0.62 mmol, 1.5 equiv.) in dry THF (2 mL) was added nBuLi (0.27 mL, 0.62 mmol, 1.5 equiv.). After 15 minutes stirring, the mixture was cooled to -78°C. In another reactor, aldehyde ***cis*-II-7** (150 mg, 0.41 mmol, 1 equiv.) was solubilised in dry THF and stirred with 4 Å molecular sieves. The aldehyde solution was cooled to -78°C and transfered dropwise *via* a canula in the ylide containing reactor. The resulting mixture was slowly allowed to warm to -60°C (4 h), quenched by addition of a THF/water 9:1 solution (10 mL), and allowed to warm to room temperature. The resulting heterogeneous mixture was extracted with diethylether (3x10 mL), washed brine (10 mL) and dried over MgSO₄. Removal of solvent afford 180 mg of a crude product which was purified by column chromatography (5% ethyl acetate, 1% triethylamine in cyclohexane) to afford 42 mg (19 %, colourless oil) of pure desired product.

The obtained ¹H NMR, ¹³C NMR and ¹⁹F NMR spectra comply with the structure of the compound.

**MS** (ESI/positive mode) : *m*/*z* (relative abundance) ; 741.07 [2M+Na]⁺ (100), 735.87 [2M+H₂O]⁺ (52), 359.47 [M+H]⁺ (10)

**IR** (film) : 3436, 2926, 1799, 1736, 1369, 1282, 1157, 1121, 1100, 772 cm⁻¹

| | | |
|---|---|---|
| **Elemental Analysis** | Calculated | C 56.81 % ; H 7.29 % ; N 3.90 % |
| | Found | C 56.90 % ; H 7.30 % ; N 3.73 % |

### ► Methyl 1-amino-2-vinyl-2-fluorocyclopropyl carboxylate with a cis configuration (cis-II-9)

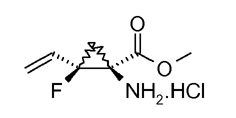

A solution of ***cis*-II-8** (252 mg, 0.7 mmol, 1 equiv.) in EtOAc (30 mL) at 0°C was saturated with HCl(g). Consumption of starting material was monitored by TLC. Solvent was evaporated and the solid obtained was washed with diethylether to afford 88 mg of desired product (65%, colourless solid) which was used without further purification.

The obtained ¹H NMR, ¹³C NMR and ¹⁹F NMR spectra comply with the structure of the compound.

### ► Methyl 1-(N,N-diterbutyloxycarbonyl)amino-2-(2'-ethoxycarbonyl)vinyl-2-fluoro cyclopropyl carboxylate with a cis configuration (cis-II-10)

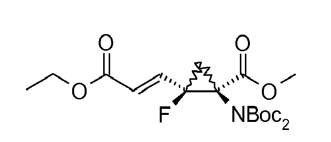

A solution of aldehyde ***cis*-II-7** (380 mg, 1.05 mmol, 1 equiv.), lithium bromide (184 mg, 2.1 mmol, 2 equiv.) and triethyl phosphonoacetate (0.425 mL, 2.1 mmol, 2 equiv.) in THF (5 mL) was stirred at room temperature until lithium bromide was totally dissolved. Triethylamine (0.293 mL, 2.1 mmol, 2 equiv.) was added dropwise and the mixture was stirred overnight. It was then filtered through a pad of celite, concentrated and purified by column chromatography (10% ethyl acetate, 1% triethylamine in cyclohexane) to afford 414 mg (91 %, colourless oil) of pure desired product.

The obtained ¹H NMR, ¹³C NMR and ¹⁹F NMR spectra comply with the structure of the compound.

**MS** (ESI/positive mode) : *m*/*z* (relative abundance) ; 885.07 [2M+Na]⁺ (100), 879.93 [2M+H₂O]⁺ (54), 454.07 [M+Na]⁺ (20), 432.27 [M+H]⁺ (16)

**IR** (film) : 3440, 2981, 2933, 1800, 1732, 1369, 1278, 1157, 1120, 1099, 853, 766 cm⁻¹

### ► trans-Methyl 1-(N,N-diterbutyloxycarbonyl)-2-(2'-ethoxycarbonyl) vinyl-2-fluoro cyclopropyl carboxylate with a trans configuration (trans-II-I0)

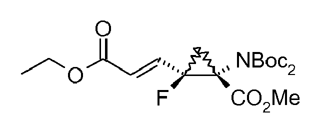

A solution of aldehyde ***trans*-II-7** (639 mg, 1.77 mmol, 1 equiv.), lithium bromide (308 mg, 3.55 mmol, 2 equiv.) and triethyl phosphonoacetate (0.705 mL, 3.55 mmol, 2 equiv.) in THF (10 mL) was stirred at room temperature until lithium bromide was totally solubilised. Triethylamine (0.495 mL, 3.55 mmol, 2 equiv.) was added and the mixture was stirred 3 days. It was then filtered through a pad of celite, concentrated and purified by column chromatography (10% ethyl acetate, 1% triethylamine in cyclohexane) to afford 211 mg (91 %, colourless oil) of pure desired product.

The obtained ¹H NMR, ¹³C NMR and ¹⁹F NMR spectra comply with the structure of the compound.

**MS** (ESI/positive mode) : *m*/*z* (relative abundance) ; 470.03 [M+K]⁺ (39), 454.06 [M+Na]⁺ (100)

**IR** (film) : 3437, 2980, 2930, 1747, 1722, 1370, 1279, 1164, 1123, 1033, 854, 786 cm⁻¹

| | | |
|---|---|---|
| **Elemental Analysis** | Calculated | C 55.68 % ; H 7.01 % ; N 3.25 % |
| | Found | C 55.73 % ; H 6.75 % ; N 3.33 % |

### ► Methyl 1-(N,N-diterbutyloxycarbonyl)amino-2-(2'-hydroxycarbonyl) vinyl-2-fluoro cyclopropyl carboxylate with a cis configuration (cis-II-11)

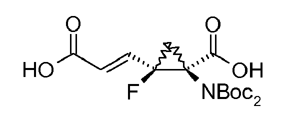

To a solution of ***cis*-II-I0** (414 mg, 0.96 mmol, 1 equiv.) in THF:H₂O (3:4) was added lithium hydroxide 1.2N solution in water (16 mL) and the resulting mixture was stirred at room temperature until starting material was consumed (NMR monitoring). The mixture was quenched at 0°C by dropwise addition of a hydrochloride 4N solution until pH = 2. Organic layer was separated and aqueous layer was extracted with EtOAc (3x10 mL). Organic layers were assembled, washed with brine and dried over MgSO₄. Solvent was removed to furnish pure desired product quantitatively (colourless oil).

The obtained ¹H NMR, ¹³C NMR and ¹⁹F NMR spectra comply with the structure of the compound.

**MS** (ESI/positive mode) : *m*/*z* (relative abundance) ; 801.00 [2M+Na]⁺ (48), 795.87 [2M+H₂O]⁺ (100)

**IR** (KBr) : 3216, 3112, 2982, 1791, 1730, 1708, 1693, 1365, 1304, 1264, 1161, 1113, 991, 936, 854, 826, 783, 676 cm⁻¹

| | | |
|---|---|---|
| **Elemental Analysis** | Calculated | C 52.44 % ; H 6.21 % ; N 3.60 % |
| | Found | C 52.41 % ; H 6.32 % ; N 3.58 % |

### ► Methyl 1-(N,N-diterbutyloxycarbonyl)amino-2-(2'-ethoxycarbonyl)ethyl-2-fluoro cyclopropyl carboxylate with a cis configuration (cis-II-12)

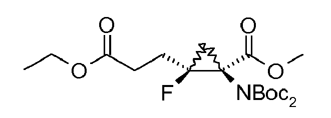

A suspension of ***cis*-II-I0** (1.9 g, 4.4 mmol) and catalytic amount of palladium on carbon in THF (20 mL) was saturated in hydrogen. This suspension was stirred at room temperature until starting material was consumed and was filtered off. Solvent was removed and the crude product which was purified by column chromatography (5% ethyl acetate, 1% triethylamine in cyclohexane) to afford 1.62 g (86 %, colourless oil) of pure desired product.

The obtained ¹H NMR, ¹³C NMR and ¹⁹F NMR spectra comply with the structure of the compound.

**MS** (ESI/positive mode) : *m*/*z* (relative abundance) ; 889.07 [2M+Na]⁺ (83), 456.00 [M+Na]⁺ (100)

**IR** (film) : 3437, 2981, 1736, 1368, 1278, 1254, 1158, 1119, 1028, 855, 785 cm⁻¹

| | | |
|---|---|---|
| **Elemental Analysis** | Calculated | C 55.42 % ; H 7.44 % ; N 3.23 % |
| | Found | C 55.69 % ; H 7.72 % ; N 3.14 % |

### ► Methyl 1-(N,N-diterbutyloxycarbonyl)amino-2-(2'-ethoxycarbonyl)ethyl-2-fluoro cyclopropyl carboxylate with a trans configuration (trans-II-12)

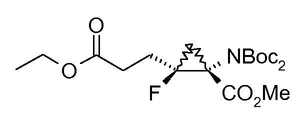

The same procedure as described above for preparing ***cis*-II-12** was applied to ***trans-*II-10** to furnish compound ***trans*-II-12** in 87% yield as a colourless oil.

The obtained ¹H NMR, ¹³C NMR and ¹⁹F NMR spectra comply with the structure of the compound.

**MS** (ESI/positive mode) : *m*/*z* (relative abundance) ; 471.96 [M+K]⁺ (29), 455.99 [M+Na]⁺ (100), 451.00 [M+H₂O]⁺ (46)

| | | |
|---|---|---|
| **Elemental Analysis** | Calculated | C 55.42 % ; H 7.44 % ; N 3.23 % |
| | Found | C 55.59 % ; H 7.69 % ; N 3.53 % |

### ► 1-(N,N-diterbutyloxycarbonyl)amino-2-(2'-hydroxycarbonyl)ethyl-2-fluoro cyclopropyl carboxylic acid with a cis configuration (cis-II-13)

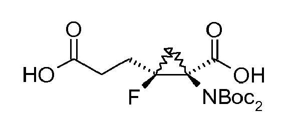

A solution of ***cis*-II-12** (1.48 g, 3.4 mmol, 1 equiv.) and lithium hydroxide (1.63 g, 68 mmol, 20 equiv.) in THF:H₂O 2:1 (40 mL) was heated at reflux overnight. Organic solvent was removed under vacuo. The resulting aqueous solution was washed with diethylether and acidified to pH = 1. Aqueous layer was then continuously extracted 5 hours with diethylether. Solvent was removed and the resulting yellow oily solid was recristallised from Et₂O/Pentane to furnish pure desired product (white solid).

The obtained ¹H NMR, ¹³C NMR and ¹⁹F NMR spectra comply with the structure of the compound.

**MS** (ESI/positive mode) : *m*/*z* (relative abundance) ; 390.93 [M+H]⁺ (100)

**IR** (KBr) : 3407, 2987, 2936, 1728, 1651, 1415, 1286, 1223, 1164, 1060, 903, 834, 797, 678, 630 cm⁻¹

| | | |
|---|---|---|
| **Elemental Analysis** | Calculated | C 52.17 % ; H 6.70 % ; N 3.58 % |
| | Found | C 51.82 % ; H 6.75 % ; N 3.36 % |

### ► cis-II-14

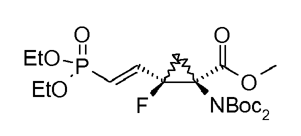

A solution of aldehyde ***cis*-II-7** (2.48 g, 6.86 mmol, 1 equiv.), lithium bromide (1.2 g, 13.7 mmol, 2 equiv.) and tetraethylmethylenediphosphonate (prepared according to Aboujaoude, E.E. et al. Tetrahedron Lett. 1985, 26, 4435) (3.4 mL, 13.7 mmol, 2 equiv.) in THF (12 mL) was stirred at room temperature until lithium bromide was totally solubilised. Triethylamine (1.91 mL, 13.7 mmol, 2 equiv.) was added dropwise and the mixture was stirred overnight. It was then filtered through a pad of celite, concentrated and purified by column chromatography (10% ethyl acetate, 1% triethylamine in cyclohexane) to afford 2.67 g (79 %, colourless oil) of pure desired product.

The obtained ¹H NMR, ¹³C NMR, ¹⁹F NMR and ³¹P NMR spectra comply with the structure of the compound.

**MS** (ESI/positive mode) : *m*/*z* (relative abundance) ; 518.07 [M+Na]⁺ (19), 512.93 [M+H₂O]⁺ (100), 495.80 [M+H]⁺ (17)

**IR** (film) : 3421, 2983, 2930, 1732, 1644, 1229, 1161, 1045, 959, 814 cm⁻¹

| | | |
|---|---|---|
| **Elemental Analysis** | Calculated | C 50.91 % ; H 7.12 % ; N 2.83 % |
| | Found | C 51.31 % ; H 6.85 % ; N 3.12 % |

### ► trans-II-14

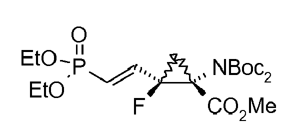

The same procedure as described above was applied to aldehyde ***trans*-II-7** to furnish compound ***trans*-II-14** in 59% yield as a colourless oil.

The obtained ¹H NMR, ¹³C NMR, ¹⁹F NMR and ³¹P NMR spectra comply with the structure of the compound.

**MS** (ESI/positive mode) : *m*/*z* (relative abundance) ; 533.90 [M+K]⁺ (19), 518.00 [M+Na]⁺ (40), 512.81 [M+H₂O]⁺ (100)

**IR** (film) : 3439, 2983, 2932, 1797, 1746, 1370, 1280, 1250, 1158, 1124, 1027, 970, 854, 787 cm⁻¹

### ► cis-II-15

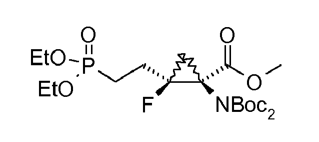

A suspension of ***cis*-II-14** (2.2 g, 4.4 mmol) and catalytic amount of palladium on carbon in THF (20 mL) was saturated in hydrogen. This suspension was stirred at room temperature until starting material was consumed and palladium on carbon was flitered off. Solvent was removed and the crude product was purified by column chromatography (5% ethyl acetate, 1% triethylamine in cyclohexane) to afford 1.12 g (51 %, colourless oil) of pure desired product.

The obtained ¹H NMR, ¹³C NMR, ¹⁹F NMR and ³¹P NMR spectra comply with the structure of the compound.

**MS** (ESI/positive mode) : *m*/*z* (relative abundance) ; 498.04 [M+H]⁺ (100)

**IR** (film) : 3452, 2982, 2935, 1798, 1735, 1439, 1368, 1277, 1253, 1219, 1162, 1120, 1097, 1057, 1028, 965, 853, 822, 787 cm⁻¹

### ► trans-II-15

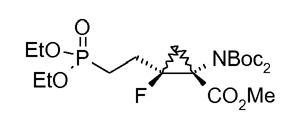

The same procedure as described above for the preparation of ***cis*-II-15** was applied to ***trans*-II-14** to furnish compound ***trans*-II-15** in 83 % yield as a colourless oil.

The obtained ¹H NMR, ¹³C NMR and ¹⁹F NMR spectra comply with the structure of the compound.

**MS** (ESI/positive mode) : *m*/*z* (relative abundance) ; 536.03 [M+K]⁺ (30), 520.13 [M+Na]⁺ (86), 514.94 [M+H₂O]⁺ (100)

### ► cis-II-16

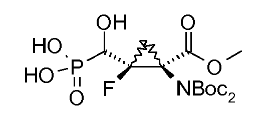

To a solution under argon of aldehyde ***cis*-II-7** (1 equiv.) and triethylamine (0.25 equiv.) in dry THF (0.6 M) was added diethylphosphonate dropwise (1.1 equiv.). The mixture was stirred at room temperature until starting material was consumed (TLC monitoring). Solvent was removed, the resulting oily product was taken up in a 1:1 mixture of diethyl ether/ethyl acetate, washed with brine, dried over MgSO₄ and concentrated. Resulting crude product was purified by column chromatography (10% ethyl acetate, 1% triethylamine in cyclohexane) to afford pure desired product as a single diastereoisomer.

The obtained ¹H NMR, ¹³C NMR, ¹⁹F NMR and ³¹P NMR spectra comply with the structure of the compound.

**MS** (ESI/positive mode) : *m*/*z* (relative abundance) ; 538.00 [M+K]⁺ (33), 521.87 [M+Na]⁺ (41), 500.07 [M+H]⁺ (100)

**IR** (film) : 3439, 2982, 2934, 1794, 1734, 1439, 1369, 1280, 1251, 1162, 1125, 1103, 1051, 1025, 972, 785, 733 cm⁻¹

| | | |
|---|---|---|
| **Elemental Analysis** | Calculated | C 48.09 % ; H 7.06 % ; N 2.80 % |
| | Found | C 47.86 % ; H 7.15 % ; N 2.77 % |

### ► trans-II-16

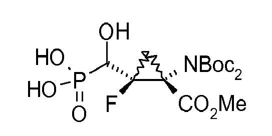

The same procedure as described above for the preparation of ***cis*-II-16** was applied to ***trans*-II-7** to furnish compound ***trans*-II-16** in 83 % yield as a colourless oil.

The obtained ¹H NMR, ¹³C NMR, ¹⁹F NMR and ³¹P NMR spectra comply with the structure of the compound.

**MS** (ESI/positive mode) : *m*/*z* (relative abundance) ; 522.06 [M+Na]⁺ (77), 516.87 [M+ H₂O]⁺ (41), 499.77 [M+H]⁺ (100)

**IR** (film) : 3342, 2989, 1748, 1456, 1370, 1251, 1161, 1099, 1026, 762, 668 cm⁻¹

| | | |
|---|---|---|
| **Elemental Analysis** | Calculated | C 48.09 % ; H 7.06 % ; N 2.80 % |
| | Found | C 48.12 %; H 7.12 %; N 2.81 % |

### ► Methyl 1-(N,N-diterbutyloxycarbonyl)-2-(methylsulfonyloxymethyl)-2-fluoro cyclopropyl carboxylate (II-17)

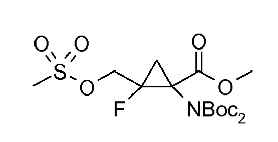

To a stirred solution of alcohol **II-6** (644 mg, 1.77 mmol, 1 equiv.) and triethylamine (0.21 mL, 3.63 mmol, 2.05 equiv.) at 0°C in Et₂O (10 mL) was added dropwise methansulfonyl chloride (0.50 mL, 2.66 mmol, 1.5 equiv.). The resulting suspension was allowed to warm to room temperature under stirring until completion (TLC monitoring). The reaction was then quenched by addition of water (5 mL) and brine was added (5 mL). Organic layer was extracted with Et₂O (3x10 mL), dried over MgSO₄ and concentrated. The resulting crude product was purified by column chromatography (5-10% ethyl acetate, 1% triethylamine in cyclohexane) to afford 752 mg (96 %, colourless oil) of pure desired product.

The obtained ¹H NMR, ¹³C NMR and ¹⁹F NMR spectra comply with the structure of the compound.

**MS** (ESI/positive mode) : *m*/*z* (relative abundance) ; 904.93 [2M+Na]⁺ (74), 479.87 [M+K]⁺ (38), 463.93 [M+Na]⁺ (83), 458.87 [M+H₂O]⁺ (100), 329.73 (96), 285.80 (54), 241.93 (72)

**IR :** 3422, 2981, 2934, 1797, 1734, 1368, 1279, 1255, 1176, 1121, 1102, 965, 851, 824, 771, 528 cm⁻¹

| | | |
|---|---|---|
| **Elemental Analysis** | Calculated | C 46.25 % ; H 6.39 % ; N 3.17 % |
| | Found | C 46.16 % ; H 6.26 % ; N 3.22 % |

### ► Methyl 1-(N-terbutyloxycarbonyl)-2-bromomethyl-2-fluoro cyclopropyl carboxylate (II-18)

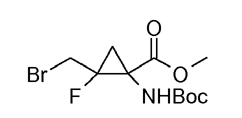

In a micro-wave reactor was stirred mesylated alcool **II-17** (457 mg, 1.03 mmol, 1 equiv.) and lithium bromide (359 mg, 4.14 mmol, 4 equiv.) in dry THF (5 mL) until lithium bromide was totally solubilised. The mixture was then irradiated under micro-wave (220 watt, 2.0 bar, 100°C) for 30 minutes. The resulting mixture was directly purified by column chromatography (5-10% ethyl acetate in cyclohexane) to afford 211 mg (65 %, brown oil) of pure desired product.

The obtained ¹H NMR, ¹³C NMR and ¹⁹F NMR spectra comply with the structure of the compound.

**MS** (CI) : *m*/*z* (relative abundance) ; 326.00 [M]⁺ (53), 270.00 (100).

**IR** (film) : 3370, 2925, 1726, 1504, 1438, 1368, 1327, 1250, 1166, 1076, 1049, 773 cm⁻¹

### ► Methyl 1-(N-trifluoromethylcarbonyl)-2-(methylsulfonyloxymethyl)-2-fluoro cyclopropyl carboxylate (II-19)

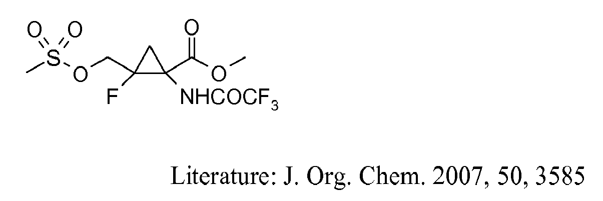

A solution of **II-17** (1 equiv.) in a hydrochloric acid saturated ethylacetate solution was stirred at room temperature for two hours (TLC monitoring). Solvent was removed and the resulting solid was suspended in dichloromethane. Trifluoroacetic anhydride (1 equiv.) was added dropwise and the mixture was stirred at room temperature for one hour. Dichloromethane was evaporated and remaining trifuoroacetic acid was coevaporated with toluene. Resulting product was used without further purification.

The obtained ¹H NMR, ¹³C NMR and ¹⁹F NMR spectra comply with the structure of the compound.

**IR** (film) : 3419, 2923, 1732, 1441, 1357, 1173, 1094, 1047, 962, 811 cm⁻¹

### ► Methyl 1-(N-trifluoromethylcarbonyl)-2-bromomethyl-2-fluoro cyclopropyl carboxylate (II-20)

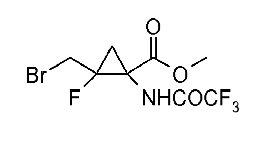

The same procedure as described above for preparing **II-19** was applied to **II-18** to furnish compound **II-20** in 83 % yield as a colourless oil.

The obtained ¹H NMR and ¹⁹F NMR spectra comply with the structure of the compound.

### ► Methyl 1-(N,N-diterbutyloxycarbonyl)-2-iodomethyl-2-fluoro cyclopropyl carboxylate (II-21)

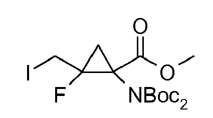

To a solution of mesylated alcohol **II-17** (4.30 g, 9.74 mmol, 1 equiv.) in dry acetone (200 mL) was added sodium iodide (11.6 g, 77.92 mmol, 8 equiv.) and tetra *n-*butylammonium iodide (1.80 g, 4.87 mmol, 0.5 equiv.). The resulting mixture was stirred and heated at reflux until completion (¹⁹F NMR and TLC monitoring). Solvent was removed, the resulting oily crude product was taken up in ethylacetate, washed with Na₂S₂O₃ 10% aqueous solution, water, brine and dried over MgSO₄. Removal of solvent afford 5.17 g of crude product which was purified by column chromatography (5% ethyl acetate, 1% triethylamine in cyclohexane) to afford 3.18 g (68 %, orange oil) of pure desired product.

The obtained ¹H NMR, ¹³C NMR and ¹⁹F NMR spectra comply with the structure of the compound.

**MS** (ESI/positive mode) : *m*/*z* (relative abundance) ; 968.73 [2M+Na]⁺ (100), 496.07 [M+Na]⁺ (27)

**IR** (film) : 3412, 2979, 1799, 1731, 1436, 1368, 1279, 1254, 1156, 1116, 853, 785 cm⁻¹

| | | |
|---|---|---|
| **Elemental Analysis** | Calculated | C 40.60 % ; H 5.32 % ; N 2.96 % |
| | Found | C 40.62 % ; H 5.34 % ; N 2.88 % |

### ► cis-II-22

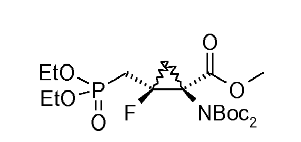

A mixture, under argon, of **II-21** (238 mg, 0.50 mmol, 1 equiv.) in distilled triethylphosphite (3 mL) was heated at 120°C in a sealed tube until complete conversion (¹⁹NMR and TLC monitoring). The excess of phosphite was then removed under vacuo without heating and the resulting oily crude product was purified by column chromatography (30% ethyl acetate, 1% triethylamine in cyclohexane) to afford 91 mg (37 %, colourless oil) of pure desired product.

The obtained ¹H NMR, ¹³C NMR, ¹⁹F NMR and ³¹P NMR spectra comply with the structure of the compound.

**MS** (ESI/positive mode) : *m*/*z* (relative abundance) ; 988.73 [2M+Na]⁺ (100), 983.80 [2M+H₃O]⁺ (76), 585.13 [M+TEA+H]⁺ (48), 506.27 [M+Na]⁺ (32), 484.13 [M+H]⁺ (46)

**IR** (film) : 3438, 2982, 2924, 1723, 1444, 1369, 1247, 1217, 1024, 964, 795 cm⁻¹

| | | |
|---|---|---|
| **Elemental Analysis** | Calculated | C 49.69 % ; H 7.30 % ; N 2.90 % |
| | Found | C 49.85 % ; H 7.32 % ; N 2.63 % |

### ► cis-II-23

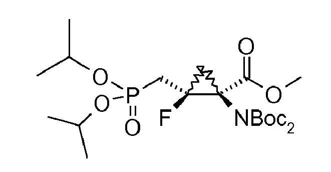

A mixture under argon of ioded product **II-21** (74 mg, 0.16 mmol, 1 equiv.) in distilled triisopropylphosphite (1 mL) was heated at 150°C in a sealed tube until complete conversion (¹⁹NMR and TLC monitoring). The excess of phosphite was then removed under vacuo without heating and the resulting oily crude product was purified by column chromatography (30% ethyl acetate, 1% triethylamine in cyclohexane) to afford 23 mg (37 %, colourless oil) of pure desired product.

The obtained ¹H NMR, ¹³C NMR, ¹⁹F NMR and ³¹P NMR spectra comply with the structure of the compound.

**MS** (ESI/positive mode) : *m*/*z* (relative abundance) ; 534.13 [M+Na]⁺ (37), 512.13 [M+H]⁺ (100)

| | | |
|---|---|---|
| **Elemental Analysis** | Calculated | C 51.66 % ; H 7.68 % ; N 2.74 % |
| | Found | C 51.79 % ; H 7.63 % ; N 2.84 % |

### ► Coupling product of L-Ala-OMe and cis-II-4 (cis-II-24)

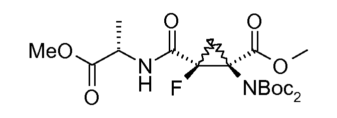

Compound ***cis*-II-4** (146 mg, 0.38 mmol, 1 equiv.), alanine methyl ester hydrochloride (83 mg, 0.43 mmol, 1.1 equiv.), HOBT (78 mg, 0.56 mmol, 1.5 equiv.) and *N*-methylmorpholine (130 µL, 1.18 mmol, 3.05 equiv.) were solubilised in dry dichloromethane (10 mL). EDCI (78 mg, 0.41 mmol, 1.05 equiv.) was added and the mixture was stirred at room temperature for 36 hours. The reaction was quenched with water (10 mL), extracted with dichloromethane (3 x 10 mL), organic layer was washed with brine (10 mL), dried over MgSO₄ and concentrated under reduced pressure. The oily crude product was purified on silica gel (10% EtOAc in cyclohexane) to furnish 158 mg (90 %) of ***cis*-II-24** (yellow oil) as a mixture of diastereoisomers (1:1).

The obtained ¹H NMR, ¹³C NMR and ¹⁹F NMR spectra comply with the structure of the compound.

**MS** (ESI/positive mode) : *m*/*z* (relative abundance) ; 302.27 (13), 485.27 [M+Na]⁺ (100), 947.07 [2M+Na]⁺ (60)

**IR** (film) : 3348, 2982, 1796, 1746, 1714, 1538, 1455, 1369, 1315, 1276, 1221, 1127, 1026, 865, 835, 759, 667, 465 cm⁻¹

### ► Coupling product of L-Ala-OMe and trans-II-4 (trans-II-24)

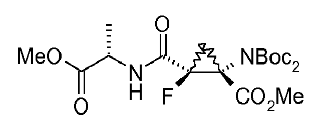

Compound ***trans*-II-4** (54 mg, 0.14 mmol, 1 equiv.), alanine methyl ester hydrochloride (31 mg, 0.16 mmol, 1.1 equiv.), HOBT (21 mg, 0.15 mmol, 1.05 equiv.) and *N*-methylmorpholine (48 µL, 0.44 mmol, 3.05 equiv.) were solubilised in dry dichloromethane (5 mL). EDCI (29 mg, 0.15 mmol, 1.05 equiv.) was added and the mixture was stirred at room temperature for 36 hours. The reaction was quenched with water (10 mL), extracted with dichloromethane (3 x 10 mL), organic layer was washed with brine (10 mL), dried over MgSO₄ and concentrated under reduced pressure. The oily crude product was purified on silica gel (10% EtOAc in cyclohexane) to furnish 51 mg (77 %) of ***trans*-II-24** (yellow oil) as a mixture of diastéréoisomers (1:1).

The obtained ¹H NMR, ¹³C NMR and ¹⁹F NMR spectra comply with the structure of the compound.

**MS** (ESI/mode positive) : *mlz* (abondance relative) ; 485,40 [M+Na]⁺ (57) ; 947,27 [2M+Na]⁺ (100)

### ► cis-II-25

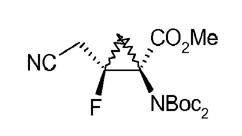

Alcohol ***cis*-II-6** (554 mg, 1.52 mmol) was dissolved in anhydrous THF (5 mL) under an argon atmosphere. The mixture was cooled down to 0°C and PPh₃ (786 mg, 3.0 mmol) was added as one portion. A solution of DBAD (702 mg, 3.0 mmol) in anhydrous THF (2 mL) was added dropwise and the mixture stirred for 30 minutes. α-Hydroxyisobutyronitrile (275 µL, 3.0 mmol) in anhydrous THF (500 µL) was added dropwise. The mixture was stirred at this temperature for 30 minutes, and was then allowed to reach room temperature and stirred for a further 12 hours. The reaction mixture was concentrated *in vacuo* and recrystallised from Et₂O/petroleum ether (50:50) to eliminate the phosphine oxide. The solid was discarded and the filtrate was concentrated and purified by column chromatography (EtOAc/cyclohexane 15:85). The compound could not be fully separated from the reduced DBAD residue and was transferred impure to the deprotection step as a yellow oily residue.

The obtained ¹H NMR, ¹³C NMR and ¹⁹F NMR spectra comply with the structure of the compound.

m/z (ES⁺): 395.07 [M+Na]⁺

### ► cis-II-26 (obtained by a Mitsunobu reaction)

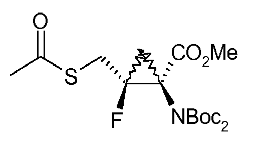

DIAD (324 µL, 1.64 mmol) was added dropwise to a stirred solution of PPh₃ (431 mg, 1.64 mmol) in anhydrous THF (5 mL) at 0°C under argon. The mixture was stirred at this temperature for 30 minutes until formation of a white precipitate of Mitsunobu betaine, and a solution of thioacetic acid (120 µL, 1.64 mmol) and alcohol **cis-II-6** (299 mg, 0.82 mmol) in anhydrous THF (3 mL) was added slowly. The reaction was stirred at 0°C for 1 hour, allowed to reach room temperature and stirred for a further hour. The solvent was removed *in vacuo* and the residue was taken up into a mixture of diethyl ether and cyclohexane (50 :50 v/v) and triturated at 0°C. The resulting white solid was filtered off and washed with Et₂O/cyclohexane. The filtrate was evaporated and the residue was purified by column chromatography (Ethyl acetate/cyclohexane, 20 :80 v/v) to afford a colorless oil (130 mg, 38% yield). The obtained ¹H NMR, ¹³C NMR and ¹⁹F NMR spectra comply with the structure of the compound.

m/z 444.20 [M+Na]⁺
microanalysis : calculated C% 51.29, H% 6.70, N% 3.32, S% 7.61, found C% 51.26, H% 6.80, N% 2.09, S% 7.41

### ► trans-II-26 (obtained by a Mitsunobu reaction)

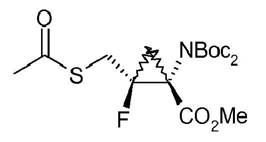

A solution of DBAD (1.113 g, 4.83 mmol) in anhydrous THF (5 mL) was added dropwise to a stirred solution of PPh₃ (1.268 g, 4.83 mmol) in anhydrous THF (5 mL) at 0°C under argon. The mixture was stirred at this temperature for 30 minutes until formation of a white precipitate of Mitsunobu betaine, and a solution of thioacetic acid (346 µL, 4.83 mmol) and alcohol ***trans*-II-6** (879 mg, 2.42 mmol) in anhydrous THF (3 mL) was added slowly. The reaction was stirred at 0°C for 1 hour, allowed to reach room temperature and stirred for a further hour. The solvent was removed *in vacuo* and the residue was taken up into a mixture of diethyl ether and cyclohexane (50:50 v/v) and triturated at 0°C. The resulting white solid was filtered off and washed with Et₂O/cyclohexane. The filtrate was evaporated and the residue was purified by column chromatography (Ethyl acetate/cyclohexane/Et₃N, 5:94.5:0.5 v/v) to afford a colorless oil (130 mg, 38% yield).

The obtained ¹H NMR, ¹³C NMR and ¹⁹F NMR spectra comply with the structure of the compound.

m/z (ES⁺) : 444.40 [M+Na]⁺, 865.07 [2M+Na]⁺

### ► cis-II-27

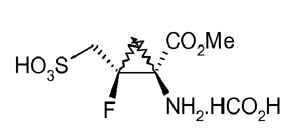

Thioacetate ***cis*-II-26** (219 mg, 0.5 mmol) was dissolved in formic acid (3 mL), and a 30% solution of aqueous hydrogen peroxyde (1.5 mL) was added slowly. The mixture was stirred at room temperature for 14 hours and monitored by ¹⁹F nmr. Upon completion the solvent was removed *in vacuo* to afford the title compound as the formate salt, as a white powder (132 mg, 95% yield).

The obtained ¹H NMR, ¹³C NMR and ¹⁹F NMR spectra comply with the structure of the compound.

m/z (ES⁺): 228.07 [M+H]⁺, 454.93 [2M+H]⁺
microanalysis : calculated C% 30.77, H% 4.43, N% 5.13, S% 11.73, found C% 28.78, H% 4.19, N% 3.79, S% 11.49

### ► trans-II-27

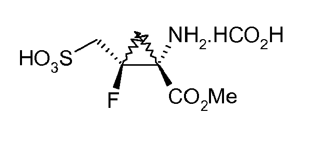

Thioacetate ***trans*-II-26** (588 mg, 1.40 mmol) was dissolved in formic acid (6 mL), and a 30% solution of aqueous hydrogen peroxyde (3 mL) was added slowly. The mixture was stirred at room temperature for 72 hours and monitored by ¹⁹F NMR. Upon completion the solvent was removed *in vacuo* and the white residue recrystallised from EtOAc/MeOH (60:40) to afford the title compound as the formate salt, as a white powder (99 mg, 26% yield).

The obtained ¹H NMR, ¹³C NMR and ¹⁹F NMR spectra comply with the structure of the compound.

m/z (ES⁻): 226.27 [M-H]⁻, 453.07 [2M-H]⁻
microanalysis : calculated C% 30.77, H% 4.43, N% 5.13, S% 11.73; found C% 29.76, H% 4.17, N% 5.27, S% 11.84

### ► trans-II-28

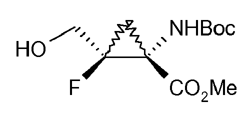

To a stirred solution of alcohol ***trans*-II-6** (349 mg, 0.96 mmol) in acetonitrile (10 mL) was added LiBr (250 mg, 2.9 mmol) as small portions. The reaction was heated to 60°C and carefully monitored by ¹⁹F NMR et TLC (cyclohexane/ EtOAC 50:50). After 7 hours, the mixture was allowed to cool down to room temperature and stirred for further 16 hours. The solvent was then removed and the residue was taken up into EtOAc (10mL). The solution was washed with water (3 x 5 mL) and brine (2 x 5mL), dried over MgSO₄ and evaporated. The yellow oily residue was purified by column chromatography (cyclohexane/ EtOAc 60:40) to afford a pure colorless oil (162 mg, 64% yield).

The obtained ¹H NMR, ¹³C NMR and ¹⁹F NMR spectra comply with the structure of the compound.

m/z (ES⁺): 263.93 [M+H]⁺, 281.13 [M+H₂O]⁺

### ► trans-II-29

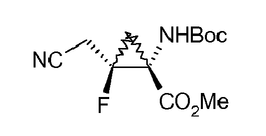

Alcohol ***trans*-II-28** (44 mg, 0.16 mmol) was dissolved in anhydrous THF (0.7 mL) under an argon atmosphere. The mixture was cooled down to 0°C and PPh₃ (84 mg, 0.32 mmol) was added as one portion. A solution of DBAD (74 mg, 0.32 mmol) in anhydrous THF (0.4 mL) was added dropwise and the mixture stirred for 30 minutes. α-Hydroxyisobutyronitrile (30 µL, 0.32 mmol) in anhydrous THF (0.5 mL) was added dropwise. The mixture was stirred at this temperature for 30 minutes, and was then allowed to reach room temperature and stirred for a further 4 hours. The reaction mixture was concentrated *in vacuo* and recrystallised from Et₂O/petroleum ether (50:50) to eliminate the phosphine oxide. The solid was discarded and the filtrate was concentrated and purified by column chromatography (EtOAc/cyclohexane 15:85) to afford a colorless oil (22 mg, 51% yield).

The obtained ¹H NMR, ¹³C NMR and ¹⁹F NMR spectra comply with the structure of the compound.

m/z (ES⁺): 295.07 [M+Na]⁺, 311.17 [M+K]⁺

### 1.3. Synthesis of the compounds of the invention of formula (I)

### ► 1-ammo-2-(2'-hydroxycarbonyl)ethyl-2-fluoro cyclopropyl carboxylic acid with a cis configuration (cis-I-1)

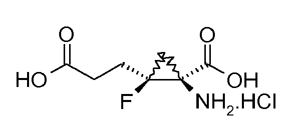

A solution of ***cis*-II-13** (135 mg, 0.345 mmol) in 1:1 solution of concentrated HCl and acetic acid was stirred 24 hours at room temperature. Solvents were removed, the resulting brown solid was taken up in water washed with ethyl acetate and lyophilised to furnish 56 mg (71%, white solid) of pure cis-FAC5 **168** as a white solid.

The obtained ¹H NMR, ¹³C NMR and ¹⁹F NMR spectra comply with the structure of the compound.

**MS** (ESI/positive mode) : *m*/*z* (relative abundance) ; 889.07 [2M+Na]⁺ (83), 456.00 [M+Na]⁺ (100)

| | | |
|---|---|---|
| **Elemental Analysis** | Calculated | C 55.42 % ; H 7.44 % ; N 3.23 % |
| | Found | C 55.43 % ; H 7.28 % ; N 3.32 % |

### ► cis-I-2

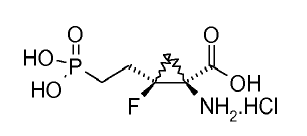

***cis*-II-15** (538 mg, 1.08 mmol) was heated at 80°C in a 1:1 mixture of acetic acid and hydrochloric acid until complete conversion (¹⁹NMR and TLC monitoring). Solvents were removed, the crude solid product was taken up in HCl 1N solution (10 mL), washed with diethylether (5 mL), dichloromethane (5 mL), lyophilised and purified on Dowex column to furnish 140 mg (53 %, colourless oil) of pure desired product.

The obtained ¹H NMR, ¹³C NMR, ¹⁹F NMR and ³¹P NMR spectra comply with the structure of the compound.

**MS** (ESI/negative mode) : *mlz* (relative abundance) ; 679.93 [3M-H]⁻ (61), [2M-H]⁻ (65), 226.20 [M-H]⁻ (100)

| | | |
|---|---|---|
| **Elemental Analysis** | Calculated | C 27.34 % ; H 4.59 % ; N 5.31 % |
| | Found | C 27.18 % ; H 4.95 % ; N 5.16 % |

### ► trans-I-2

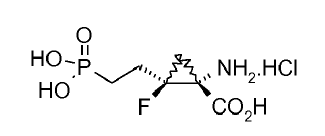

The same procedure as described above was applied to ***trans*-II-15** to furnish compound ***trans*-I-2** in 15 % yield, as a white solid.

The obtained ¹H NMR, ¹³C NMR, ¹⁹F NMR and ³¹P NMR spectra comply with the structure of the compound.

**MS** (ESI/negative mode) : *m*/*z* (relative abundance) ; 226.27 [M-H]⁻ (37), 453.20 [2M-H]⁻ (100), 679.87 [3M-H]⁻ (27)

| | | |
|---|---|---|
| **Elemental Analysis** | Calculated | C 27.34 % ; H 4.59 % ; N 5.31 % |
| | Found | C 27.16 %; H 4.95 %; N 5.01 % |

### ► cis-I-3

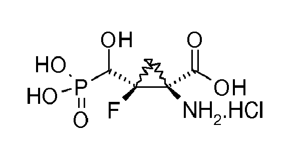

A solution of ***cis*-II-16** (1 equiv.) in a 1:1 mixture of acetic acid and concentrated hydrochloric acid (0.05 M) was heated under reflux until completion (NMR and TLC monitoring). Solvent was removed, the resulting oily product was taken up in a 1:1 mixture of diethyl ether/ethyl acetate, washed with brine, dried over MgSO₄ and concentrated. Resulting crude product was purified by column chromatography (10% ethyl acetate, 1% triethylamine in cyclohexane) to afford pure desired product as a single diastereoisomer.

The obtained ¹H NMR, ¹³C NMR, ¹⁹F NMR and ³¹P NMR spectra comply with the structure of the compound.

**MS** (ESI/negative mode) : *m*/*z* (relative abundance) ; 227.93 [M-H]⁻ (100)

| | | |
|---|---|---|
| **Elemental Analysis** | Calculated | C 22.61 % ; H 3.80 % ; N 5.27 % |
| | Found | C 22.56 % ; H 4.02 % ; N 5.31 % |

### ► trans-I-3

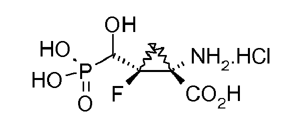

The same procedure as described above was applied to ***trans*-II-16** to furnish compound ***trans*-I-3** in 15 % yield, as a white solid.

The obtained ¹H NMR, ¹³C NMR, ¹⁹F NMR and ³¹P NMR spectra comply with the structure of the compound.

**MS** (ESI/positive mode) : *m*/*z* (relative abundance) ; 230.10 [M+H]⁺ (100)

| | | |
|---|---|---|
| **Elemental Analysis** | Calculated | C 22.61 % ; H 3.80 % ; N 5.27 % |
| | Found | C 22.58 % ; H 3.47 % ; N 4.94 % |

### ► cis-I-4

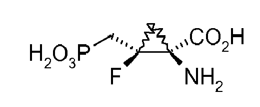

The precursor **II-22** (90 mg, 0.19 mmol) was heated at 80°C in a 1:1 mixture of acetic acid and hydrochloric acid until complete conversion (¹⁹NMR and TLC monitoring). Solvents were removed, the crude solid product was taken up in HCl 1N solution (10 mL), washed with diethylether(5 mL), dichloromethane (5 mL), lyophilised and purified on Dowex column to furnish 41 mg (88 %, brown solid) of pure desired product.

The obtained ¹H NMR, ¹³C NMR, ¹⁹F NMR and ³¹P NMR spectra comply with the structure of the compound.

**MS** (ESI/negative mode) : *m*/*z* (relative abundance); 212.13 [M-H]⁻ (100) ; (ESI/positive mode) : 213.93 [M+H]⁺ (88), 230.93 [M+H₃O]⁺ (100)

| | | |
|---|---|---|
| **Elemental Analysis** | Calculated | C 28.18 % ; H 4.26 % ; N 6.57 % |
| | Found | C 28.34 % ; H 4.34 % ; N 6.32 % |

The two enantimers of ***cis-*I-4 (*cis*-I-4a** and ***cis*-I-4b)** were prepared according to the same protocole from ***cis*-II-4a** and ***cis*-II-4b.**

### ► cis-I-5

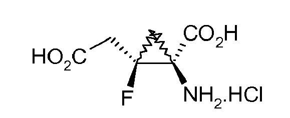

Impure nitrile ***cis*-II-25** (92 mg, 0.25 mmol) was dissolved in 6N HCl (4 mL). The reaction was heated up to 80°C and stirred over 24 hours. The solvent was then *removed in vacuo*, and the chlorhydrate residue purified over a Dowex 1X4-400 anion exchange resin column (elution: gradient of AcOH 0.05M to 0.5M). Fractions revealing with ninhydrin were combined and freeze-dried to give a white solid (14 mg).

The obtained ¹H NMR, ¹³C NMR and ¹⁹F NMR spectra comply with the structure of the compound.

m/z (ES⁺): 178.13 [M+H]⁺, 354.96 [2M+H]⁺
microanalysis : calculated C% 33.74, H% 4.25, N% 6.56, found C% 33.59, H% 4.31, N% 6.58

### ► trans-I-5

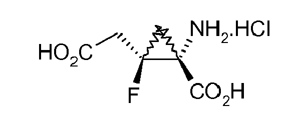

Nitrile ***trans*-II-29** (20 mg, 0.073 mmol) was dissolved in 1N HCl (2 mL). The reaction was heated up to 80°C and stirred over 24 hours. The solvent was then removed *in vacuo*, the residue was triturated in Et₂O and filtered off. The white powder was taken up into water and freeze-dried to give a white solid (8 mg, 52% yield).

The obtained ¹H NMR, ¹³C NMR and ¹⁹F NMR spectra comply with the structure of the compound.

m/z (ES⁺) : 159.13 [M+H-F]⁺

Microanalysis: calculated C% 33.74, H% 4.25, N% 6.56, found C% 33.44, H% 3.95, N% 6.60.

### ► cis-I-6

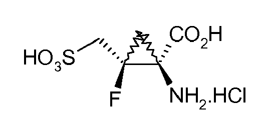

Ester ***cis*-II-27** (96 mg, 0.35 mmol) was dissolved in 6N HCl (5 mL). The reaction was heated up to 80°C and stirred over 18 hours. The solvent was then removed *in vacuo,* and the chlorhydrate residue purified by water elution over a Dowex 50WX4-50 cation exchange resin column. Fractions revealing with ninhydrin were combined and freeze-dried to give a yellow solid. Trituration in Et₂O and filtration afforded the title compound (74 mg, yield 93%).

The obtained ¹H NMR, ¹³C NMR and ¹⁹F NMR spectra comply with the structure of the compound.

m/z (ES⁻): 212.33 [M-H]⁻, 425.07 [2M-H]⁻
microanalysis : calculated C% 24.06, H% 3.63, N% 5.61, S% 12.84, found C% 24.26, H% 3.45, N% 5.36, S% 14.03

### ►trans-I-6

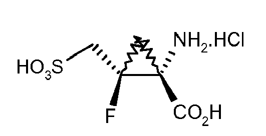

Ester ***trans*-II-27** (85 mg, 0.3 mmol) was dissolved in 6N HCl (6 mL). The reaction was heated up to 70°C and stirred over 48 hours. The solvent was then removed in *vacuo,* and the chlorhydrate residue was recrystallised from hot water to afford a white powder (58 mg, yield 77%).

The obtained ¹H NMR, ¹³C NMR and ¹⁹F NMR spectra comply with the structure of the compound.

m/z (ES⁻): 212.26 [M-H]⁻
microanalysis : calculated C% 24.06, H% 3.63, N% 5.61, S% 12.84, found C% 24.47, H% 3.46, N% 5.65, S% 12.88

### 2. Pharmacological results

The compounds according to the invention have been tested on the mGlu4 receptor at different doses (between 1nM and 1mM) in order to determine their EC50 value. This test has been carried out according to the protocol described in: C. Selvam, C. Goudet, N. Oueslati, J.-P. Pin, F. Acher J. Med. Chem. 2007, 50, 4656-4664.

The results obtained are presented on the table below:

| **Compound** | **EC50** |
|---|---|
| *cis*-I-6 | 85.6 µM |
| *cis*-I-3 | 27.4 µM |
| *cis*-I-4 | 0.34 µM |

### ABREVIATIONS

| | |
|---|---|
| Ala | Alanine |
| Boc | *tert*-Butyloxycarbonyl |
| DBAD | Di-*tert*-butylazodicarboxylate |
| DIAD | Diisobutylazodicarboxylate |
| DIBAL-H | Diisobutylaluminium hydride |
| DMAP | Dimethylaminopyridine |
| EDCI | 3-Ethyl-1(N,N-dimethyl)aminopropylcarbodiimide |
| ESI | Electrospray ionization |
| HOBT | 1-Hydroxybenzotriazole |
| HPLC | High performance liquid chromatography |
| IBX | 2-Iodoxybenzoic acid |
| IR | Infra rouge |
| MS | Mass spectrum |
| NMR | Nuclear magnetic resonance |
| Ser | Serine |
| THF | Tetrahydrofurane |
| TLC | Thin layer chromatography |

## Claims

1. Compound of the following formula (I): 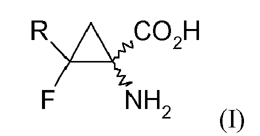 or a pharmaceutically acceptable salt, a stereoisomer or a mixture in all proportions of stereoisomers thereof, in particular a mixture of enantiomers, such as a racemic mixture,
wherein R represents a (C₁-C₆)alkanediyl or (C₁-C₆)alkenediyl group, optionally substituted by one or more groups chosen among an halogen atom, ORₐ, SR_{b}, NR_{c}R_{d}, PO(ORₑ)(OR_{f}), CO₂R_{g}, SO₂Rₕ SO₃Rᵢ, PO(OH)(CH(OH)Rₖ), CN, N₃ and NH-C(=NH)NH₂,
with Rₐ, R_{b}, R_{c} and R_{d}, representing, independently of each other, an hydrogen atom, a (C₁-C₆)alkyl group or a -CO-(C₁-C₆)alkyl group,
Rₑ, R_{f}, R_{g}, Rₕ and Rᵢ representing, independently of each other, an hydrogen atom or a (C₁-C₆)alkyl group, and
Rₖ representing an aryl or heteroaryl group, said group being optionally substituted by one or more groups selected from an halogen atom and NO₂.

2. Compound according to claim 1, **characterized in that** R is a (C₁-C₆)alkanediyl group, such as -CH₂- or -CH₂-CH₂-, substituted by a group chosen among PO₃H₂, CO₂H and SO₃H, and optionally substituted by one or more groups chosen among an halogen atom, ORₐ, SR_{b} and NR_{c}R_{d}, with Rₐ, R_{b}, R_{c} and R_{d} as defined in claim 1, and preferably chosen among OH, SH and NH₂.

3. Compound according to any one of claims 1 and 2, **characterized in that** it is chosen among:
| | | | |
|---|---|---|---|
| *cis*-I-1 | 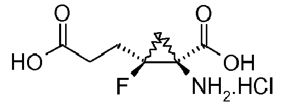 | *cis*-I-2 | 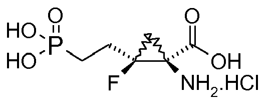 |
| *trans*-I-2 | 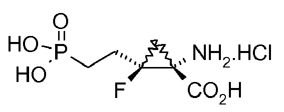 | *cis*-I-3 | 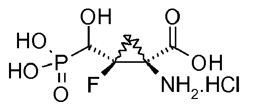 |
| *trans*-I-3 | 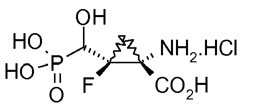 | *cis*-I-4 | 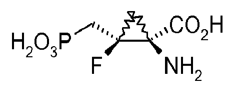 |
| *cis*-I-4a | 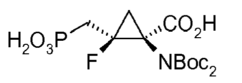 | *cis*-I-4b | 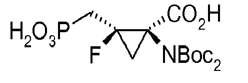 |
| *cis*-I-5 | 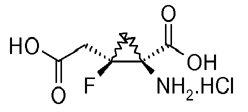 | *trans*-I-5 | 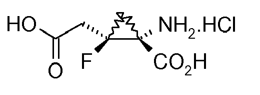 |
| *cis*-I-6 | 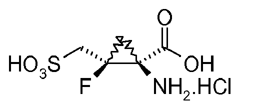 | *trans-1-6* | 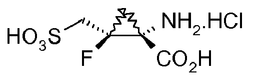 |

4. Compound according to any of claims 1 to 3 for its use as medicament, in particular in the treatment of neurological diseases, such as Alzheimer's disease, Parkinson's disease or epilepsy.

5. Pharmaceutical composition comprising at least one compound of formula (I) according to any of claims 1 to 3 and at least one pharmaceutically acceptable excipient.

6. Pharmaceutical composition according to claim 5, **characterized in that** it further comprises another active compound, useful in particular in the treatment of neurological diseases, such as acetylcholinesterase inhibitors like donezepil, galanthamine, rivastigmine, mémantine or tacrine ; monoamine oxidase inhibitors like selegiline ; catecholamin-O-methyltransferase inhibitors like entacapone ; glutamatergic inhibitors like amantadine or baclofene ; cholinergic agonists like sabcomeline ; dopaminergic agonists like pergolide, cabergoline, ropirinole or pramipexole; neuromediator analogs or precursors like L-3,4-dihydroxyphenylalanine ; and anticholinergics like trihexyphenidyl or tropatepine.

7. Pharmaceutical composition comprising:
(i) at least one compound of formula (I) according to any of claims 1 to 3, and
(ii) at least another active compound,
as a combination product for a simultaneous or separate use, or for use spread out over time.

8. Pharmaceutical composition according to claim 7, **characterized in that** the active compound is useful in the treatment of neurological diseases, and is advantageously chosen among acetylcholinesterase inhibitors like donezepil, galanthamine, rivastigmine, mémantine or tacrine ; monoamine oxidase inhibitors like selegiline ; catecholamin-O-methyltransferase inhibitors like entacapone ; glutamatergic inhibitors like amantadine or baclofene ; cholinergic agonists like sabcomeline ; dopaminergic agonists like pergolide, cabergoline, ropirinole or pramipexole; neuromediator analogs or precursors like L-3,4-dihydroxyphenylalanine ; and anticholinergics like trihexyphenidyl or tropatepine.

9. Pharmaceutical composition according to any one of claims 5 to 8 for its use as medicament, in particular in the treatment of neurological diseases, such as Alzheimer's disease, Parkinson's disease or epilepsy.

10. Process for preparing a compound of formula (Ib) corresponding to a compound of formula (I) as defined in claim 1, in which R represents -CH₂CH₂R1 with R1 representing a direct bound or a (C₁-C₄)alkanediyl, optionally substituted by one or more groups chosen among an halogen atom, such as a fluorine atom, ORₐ, SR_{b}, NRR_{d}, PO(ORₑ)(OR_{f}), CO₂R_{g}, SO₂Rₕ, SO₃Rᵢ, PO(OH)(CH(OH)Rₖ), CN, N₃ or NH-C(=NH)NH₂,
with Rₐ, R_{b}, R_{c}, R_{d}, Rₑ, R_{f}, R_{g}, Rₕ, Rᵢ and Rₖ as defined in claim 1,
comprising the following successive steps:
(a) reaction of Horner-Wadsworth-Emmons between a compound of formula (IIa): 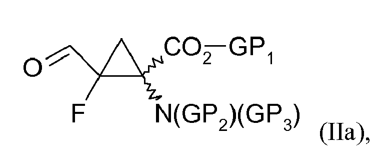 wherein GP₁ represents a O-protecting group, and GP₂ and GP₃ represent, independently of each other, a N-protecting group,
and a compound of formula (III): 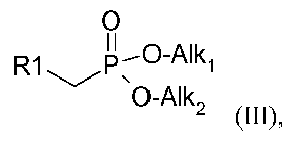 wherein Alk₁ and Alk₂ represent, independently of one another, a (C₁-C₆)alkyl group, such as an ethyl group, and R1 is as defined above,
to give a compound of formula (IV): 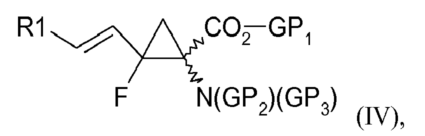 wherein R1, GP₁, GP₂ and GP₃ are as defined above,
(b) hydrogenation of the compound of formula (IV) obtained in the previous step (a) to give a compound of formula (V): 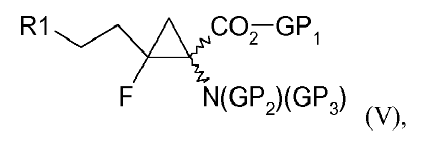 wherein R1, GP₁, GP₂ and GP₃ are as defined above,
(c) hydrolysis of the CO₂-GP₁ and N(GP₂)(GP₃) groups of the compound of formula (V) obtained in the previous step (b) to give a compound of formula (Ib): 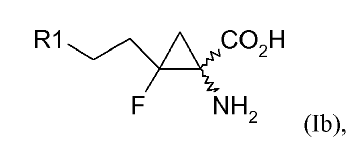 wherein R1 is as defined above, and
(d) separation of the compound (Ib) from the reaction mixture.

11. Process according to claim 10, **characterized in that** the compound of formula (IIa) is prepared according to the following successive steps:
(a1) reaction between a compound of formula (VI): 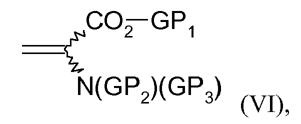 wherein GP₁, GP₂ and GP₃ are as defined in claim 10, and a compound of formula (VII),
Br₂FC-C(O)Z-Alk₅ (VII),
wherein Z represents O or NR2 with R2 representing an hydrogen atom, a (C₁-C₆)alkyl group or a (C₁-C₆)alkoxy group, and Alk₅ represents a (C₁-C₆)alkyl group, such as an ethyl group,
in the presence of ZnY¹Y² with Y¹ representing a (C₁-C₆)alkyl group and Y² representing a bromine or iodine group or a (C₁-C₆)alkyl group to give a compound of formula (IIb): 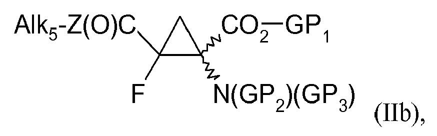 wherein Z and Alk₅ are as defined above and GP₁, GP₂ and GP₃ are as defined in claim 10,
(b1)hydrolysis of the C(O)Z-Alk₅ group of the compound of formula (IIb) obtained in the previous step (a1) to give a compound of formula (IIc): 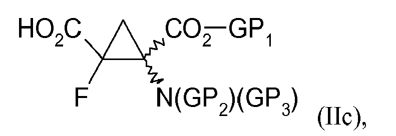 wherein GP₁, GP₂ and GP₃ are as defined in claim 10, and
(c1) reduction in aldehyde of the free carboxylic acid function of the compound of formula (IIc) obtained in the previous step (b1) to give a compound of formula (IIa).

12. Compound of the following formula (II): 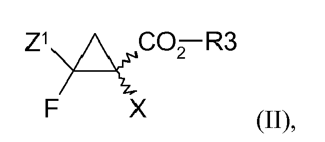 wherein:
- R3 represents an hydrogen atom or a GP₁ group as defined in claim 10,
- Z¹ represents a CHO, COOH, C(O)Z-Alk₅ or R group, with R as defined in claim 1 and with Z and Alk₅ as defined in claim 11, and
- X represents an NH₂, NHGP₂ or N(GP₂)(GP₃) group, with GP₂ and GP₃ as defined in claim 10,
provided that R3 does not represent an hydrogen atom when X represents a NH₂ group.

13. Compound according to claim 12, **characterized in that** it is chose among:
| | | | |
|---|---|---|---|
| *cis*-II-1 | 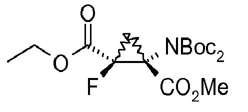 | *trans*-II-1 | 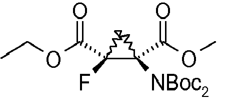 |
| II-2 | 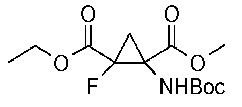 | II-3 | 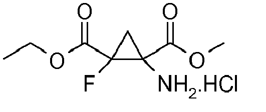 |
| *cis*-II-4 | 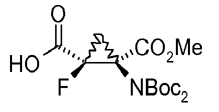 | *trans*-II-4 | 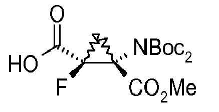 |
| *cis*-II-4a | 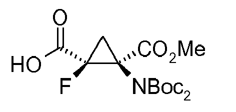 | *cis*-II-4b | 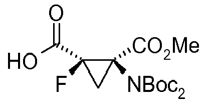 |
| *cis*-II-5 | 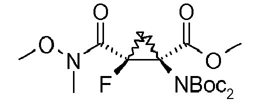 | *trans*-II-5 | 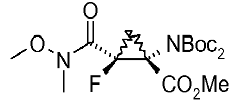 |
| *cis*-II-6 | 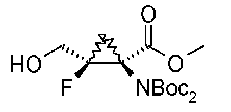 | *trans*-II-6 | 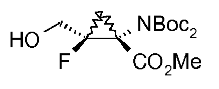 |
| *cis*-II-7 | 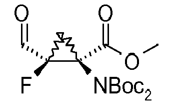 | *trans*-II-7 | 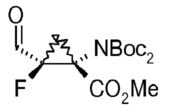 |
| *cis*-II-8 | 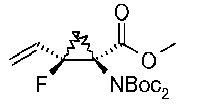 | *cis*-II-9 | 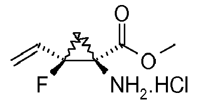 |
| *cis*-II-10 | 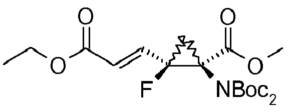 | *trans*-II-10 | 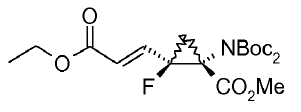 |
| *cis*-II-11 | 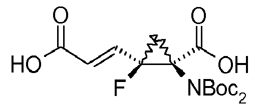 | *cis*-II-12 | 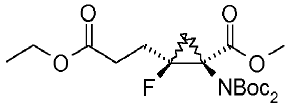 |
| *trans*-II-12 | 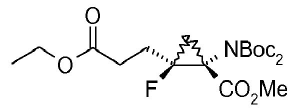 | *cis*-II-13 | 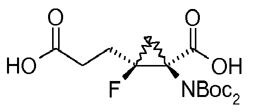 |
| *cis*-II-14 | 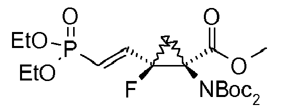 | *trans*-II-14 | 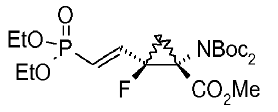 |
| *cis*-II-15 | 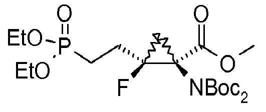 | *trans*-II-15 | 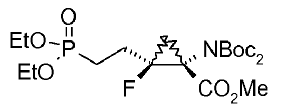 |
| *cis*-II-16 | 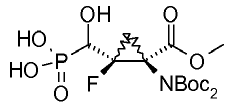 | *trans*-II-16 | 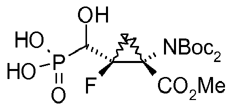 |
| II-17 | 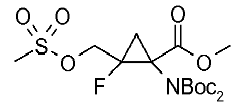 | II-18 | 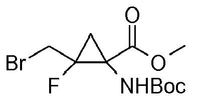 |
| II-19 | 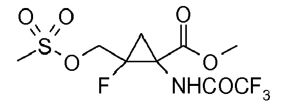 | II-20 | 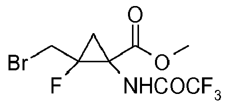 |
| II-21 | 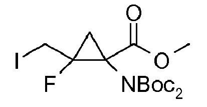 | *cis*-II-22 | 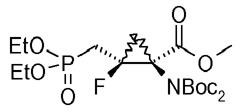 |
| *cis*-II-23 | 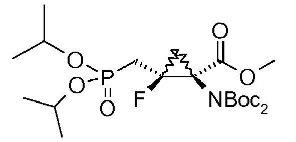 | *cis*-II-24 | 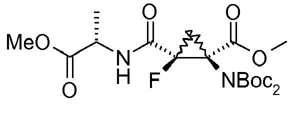 |
| *trans*-II-24 | 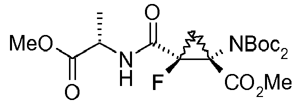 | *cis*-II-25 | 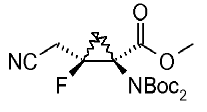 |
| *cis*-II-26 | 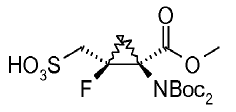 | *trans*-II-26 | 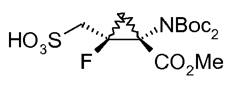 |
| *cis*-II-27 | 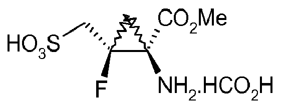 | *trans*-II-27 | 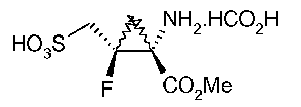 |
| *trans*-II-28 | 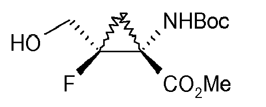 | *trans*-II-29 | 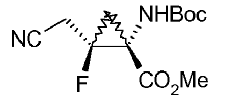 |

14. Process for preparing a compound of formula (II) as defined in claim 12 in which Z¹ represents a CHO, COOH, C(O)Z-Alk₅, CH₂OH or CH₂Hal group, R3 represents an hydrogen atom and X represents a N(GP₂)(GP3) group with GP₂ and GP₃ as defined in claim 10, Alk₅ as defined in claim 11 and Hal representing an halogen atom, comprising the following successive steps:
(a2) reaction between a compound of formula (VI) as defined in claim 11,
and a compound of formula (VII) as defined in claim 11,
in the presence of ZnY¹Y² with Y¹ and Y² as defined in claim 11 to give a compound of formula (IIb) as defined in claim 11,
(b2)optionally hydrolysis of the the C(O)Z-Alk₅ group of the compound of formula (IIb) obtained in the previous step (a2) to give a compound of formula (IIc) as defined in claim 11,
(c2) optionally reduction in aldehyde or alcohol of the free carboxylic function of the compound of formula (IIc) obtained in the previous step (b2) to give a compound of formula (IIa) as defined in claim 10 or a compound of the following formula (IId), 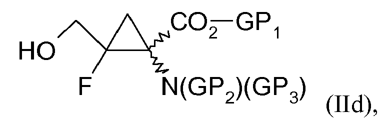 wherein GP₁, GP₂ and GP₃ are as defined in claim 10,
(d2)optionally halogenation of the alcohol moiety of the compound of formula (IId) obtained at the previous step (c2) to give a compound of the following formula (IIe), 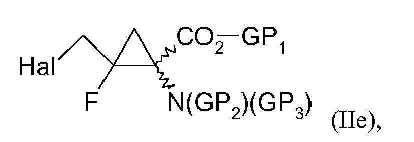 wherein GP₁, GP₂ and GP₃ are as defined in claim 10 and Hal is as defined above, and
(e2) separation of the compound of formula (IIa), (IIb), (IIc), (IId) or (IIe) from the reaction mixture.
